# EUROPEAN PATENT SPECIFICATION

(11) **EP 3 394 024 B1**
(45) Date of publication and mention of the grant of the patent: **02.08.2023**
(21) Application number: 16879515.1
(22) Date of filing: 03.06.2016
(51) Int. Cl.: C07C 59/68, C07C 59/52, A61K 47/34, C08G 67/00, C07C 69/84, C08G 64/10, C08G 64/22

(54) **POLYMERIC BIOMATERIALS DERIVED FROM PHENOLIC MONOMERS AND THEIR MEDICAL USES**
POLYMERE BIOMATERIALIEN AUS PHENOLISCHEN MONOMEREN UND DEREN MEDIZINISCHE VERWENDUNGEN
BIOMATÉRIAUX POLYMÈRES DÉRIVÉS DE MONOMÈRES PHÉNOLIQUES ET LEURS UTILISATIONS MÉDICALES

(30) Priority: 23.12.2015 WO PCT/IB2015/059967
(43) Date of publication of application: 31.10.2018
(73) Proprietor: Rutgers, The State University of New Jersey, New Brunswick, NJ 08901 (US)
(72) Inventor: BOLIKAL, Durgadas, Edison, NJ 08820 (US); KOHN, Joachim, B., Piscataway, NJ 08854 (US)
(74) Representative: Zacco GmbH
(86) International application number: PCT/US2016/035723
(87) International publication number: WO 2017/111994

(56) References cited:
- WO-A1-2015/126666
- US-A- 4 331 782
- US-A- 4 331 782
- US-A1- 2013 085 238
- US-A1- 2013 203 713
- US-A1- 2013 203 713
- US-A1- 2015 045 451
- COVELLO M ET AL: "SYNTHESIS AND PROPERTIES OF 2-HYDROXY- AND 2-ACETOXY-5-IODOBENZOIC ACID POLYESTERS OF SHORT-CHAIN ALIPHATIC POLYALCOHOLS", RICERCA SCIENTI, CONSIGLIO NAZIONALE DELLE RICERCHE, ROMA, IT, vol. 38, no. 10, 1 October 1968 (1968-10-01), pages 933-936, XP008015073, ISSN: 0035-5011

## Description

### FIELD OF THE INVENTION

The present invention relates to new classes of monomeric phenol compounds useful for preparation of biocompatible polymers and biocompatible polymers prepared therefrom, including novel biodegradable and/or bioresorbable polymers. These polymers may be adapted for radio-opacity and are useful for medical device applications and controlled release therapeutic formulations.

### BACKGROUND OF THE INVENTION

The rapidly evolving field of bioengineering has created a demand for a diverse library of different types of polymers offering a wide variety of choice of physical, mechanical, chemical and physiological properties. It is desirable that libraries of many different materials be available so that the specific polymer properties can be optimally matched with the requirements of the specific applications under development.

Examples of polymers suitable for various bioengineering applications include those described in U.S. Patent Nos. 5,099,060; 5,665,831; 5,916,998 and 6,475,477, along with the polymers described in U.S. Patent Publication Nos. 2006/0024266, 2006/0034769, 2013/0203713, 2013/085238 and 2015/0045451. There are numerous applications in which it is considered desirable for an implanted medical device to maintain its integrity and performance characteristics for extended periods of time, even under demanding mechanical conditions such as repeated mechanical flexure.

Although many types of bioresorbable and/or biodegradable polymers are known, in most of these polymers diphenolic monomers are prepared by linking two suitably protected tyrosine molecules or tyrosine analogs via an amide linkage. These amide linkages do not degrade hydrolytically under physiological conditions and therefore the monomers which have low solubility in water, dissolve very slowly. Further, due to hydrogen bonding of amide hydrogen the melt viscosity of the polymers derived from these monomers is very high, which makes thermal processing more difficult. In addition, bioresorbtion and/or biodegradation tend to alter mechanical properties in unpredictable ways that are not necessarily linearly related to each other.

Thus, there is a need for biocompatible polymers having desirable bioresorbability and biodegradability as well as good processibility under thermal conditions. There remains a need for nontoxic polyarylates having a moderate rate of bioerosion, suitable for use as tissue-compatible materials for biomedical uses.

### SUMMARY OF THE INVENTION

The present invention addresses the foregoing need by providing new monomers useful for the preparation of the desired biocompatible polymers and various types of such polymers useful for making the implantable medical devices.

The present invention broadly relates to diphenolic monomers and bioerodible polymers synthesized using such monomers.The present invention is defined by the appended claims.

### Polymers

In one aspect the present invention provides a biocompatible polymer, comprising a recurring unit of formula: 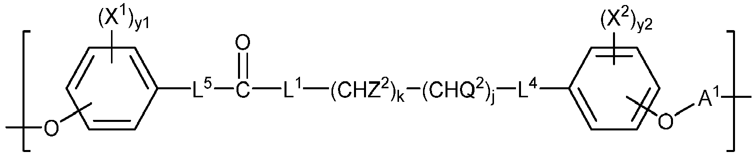 wherein:
j and k are each independently zero or an integer selected from 1 to 6, provided that j and k are not both 0;
X¹ and X² are each independently bromine (Br) or iodine (I), and y¹ and y² are each independently 0, 1, 2, 3, or 4;
L¹ is selected from the group consisting of oxygen (-O-), -R⁴-C(=O)-O-, and -O-R^{4a}-O-C(=O)-; wherein R⁴ is selected from the group consisting of C₁-C₃₀ alkylene, C₂-C₃₀ alkenylene, C₂-C₃₀ alkynylene, C₁-C₃₀ heteroalkylene, C₂-C₃₀ heteroalkenylene, C₂-C₃₀ heteroalkynylene, C₆-C₃₀ arylene, C₇-C₃₀ alkylarylene, C₈-C₃₀ alkenylarylene, C₈-C₃₀ alkynylarylene, and C₂-C₃₀ heteroarylene; and R^{4a} is selected from the group consisting of C₁-C₃₀ alkylene, C₂-C₃₀ alkenylene, C₂-C₃₀ alkynylene, C₁-C₃₀ heteroalkylene, C₂-C₃₀ heteroalkenylene, C₂-C₃₀ heteroalkynylene, C₆-C₃₀ arylene, C₇-C₃₀ alkylarylene, C₈-C₃₀ alkenylarylene, C₈-C₃₀ alkynylarylene, and C₂-C₃₀ heteroarylene;
L⁴ is selected from the group consisting of a bond, and optionally substituted phenoxy (-C₆H₄-O-);
L⁵ is selected from the group consisting of a bond and -CH₂-;
Q² at each occurrence is independently hydrogen or halogen; or two adjacent Q² form a double bond when adjacent Qs are present; or Z² and an adjacent Q² form a double bond;
Z² is hydrogen, -N(R^{x})C(=O)R⁵, -N(R^{x})COOR⁶, -C(O)OR⁷ or -C(O)NR^{x}R^{y}, or Z² and an adjacent Q² form a double bond;
wherein R⁵, R⁶, R⁷, R^{x} and R^{y} are independently at each occurrence selected from the group consisting of hydrogen, alkyl, aryl, alkylaryl, arylalkyl, heteroalkyl, and heteroalkylaryl, wherein the non-hydrogen groups contain up to 30 carbon atoms, wherein the heteroalkyl group contains from 1 to 10 heteroatoms independently selected from O, N and S, and the heteroalkylaryl group contains from 1 to 3 heteroatoms independently selected from O, N and S; and
wherein A¹ is selected from the group consisting of: 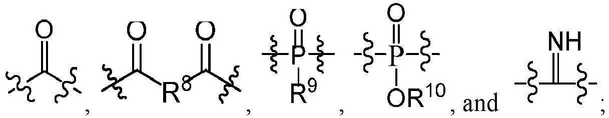
wherein R⁸ is selected from a bond, C₁-C₃₀ alkylene, C₂-C₃₀ alkenylene, C₂-C₃₀ alkynylene; C₁-C₃₀ heteroalkylene, C₂-C₃₀ heteroalkenylene, C₂-C₃₀ heteroalkynylene, C₇-C₃₀ heteroalkylarylene, C₈-C₃₀ heteroalkenylarylene, C₈-C₃₀ heteroalkynylarylene, C₇-C₃₀ alkylarylene, C₈-C₃₀ alkenylarylene, C₈-C₃₀ alkynylarylene, and C₂-C₃₀ heteroarylene; and
wherein R⁹ and R¹⁰ are each independently selected from H, C₁-C₃₀ alkyl, C₁-C₃₀ heteroalkyl, C₂-C₃₀ alkenyl, C₂-C₃₀ alkynyl, C₂-C₃₀ heteroalkenyl, and C₂-C₃₀ heteroalkynyl;
with the proviso that the biocompatible polymer is not poly(4'-hydroxyphenyethyl-2-(4-hydroxy-3,5-diiodophenyl)acetate carbonate).
In another aspect the present invention provides a biocompatible polymer, comprising a recurring unit of formula: 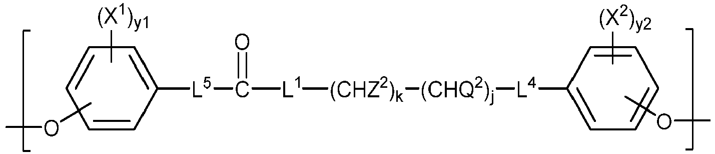 wherein:
j and k are each independently zero or an integer selected from 1 to 6, provided that j and k are not both 0;
X¹ and X² are each independently bromine (Br) or iodine (I), and y¹ and y² are each independently 0, 1, 2, 3, or 4;
L¹ is selected from the group consisting of oxygen (-O-), -R⁴-C(=O)-O-, and -O-R^{4a}-O-C(=O)-; wherein R⁴ is selected from the group consisting of C₁-C₃₀ alkylene, C₂-C₃₀ alkenylene, C₂-C₃₀ alkynylene, C₁-C₃₀ heteroalkylene, C₂-C₃₀ heteroalkenylene, C₂-C₃₀ heteroalkynylene, C₆-C₃₀ arylene, C₇-C₃₀ alkylarylene, C₈-C₃₀ alkenylarylene, C₈-C₃₀ alkynylarylene, and C₂-C₃₀ heteroarylene; and R^{4a} is selected from the group consisting of C₁-C₃₀ alkylene, C₂-C₃₀ alkenylene, C₂-C₃₀ alkynylene, C₁-C₃₀ heteroalkylene, C₂-C₃₀ heteroalkenylene, C₂-C₃₀ heteroalkynylene, C₆-C₃₀ arylene, C₇-C₃₀ alkylarylene, C₈-C₃₀ alkenylarylene, C₈-C₃₀ alkynylarylene, and C₂-C₃₀ heteroarylene;
L⁴ is selected from the group consisting of a bond, and optionally substituted phenoxy (-C₆H₄-O-);
L⁵ is selected from the group consisting of a bond and -CH₂-;
Q² at each occurrence is independently hydrogen or halogen; or two adjacent Q² form a double bond when adjacent Qs are present; or Z² and an adjacent Q² form a double bond;
Z² is hydrogen, -N(R^{x})C(=O)R⁵, -N(R^{x})COOR⁶, -C(O)OR⁷ or -C(O)NR^{x}R^{y}, or Z² and an adjacent Q² form a double bond; and
wherein R⁵, R⁶, R⁷, R^{x} and R^{y} are independently at each occurrence selected from the group consisting of hydrogen, alkyl, aryl, alkylaryl, arylalkyl, heteroalkyl, and heteroalkylaryl, wherein the non-hydrogen groups contain up to 30 carbon atoms, wherein the heteroalkyl group contains from 1 to 10 heteroatoms independently selected from O, N and S, and the heteroalkylaryl group contains from 1 to 3 heteroatoms independently selected from O, N and S.

### Monomers

In another aspect the present invention provides a diphenol compound having the formula: 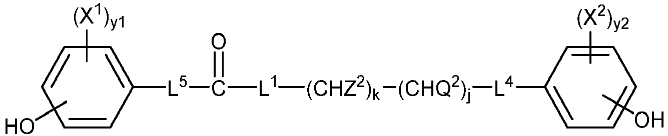 wherein:
j and k are each independently zero or an integer selected from 1 to 6, provided that j and k are not both 0;
X¹ and X² are each independently bromine (Br) or iodine (I), and y¹ and y² are each independently 0, 1, 2, 3, or 4;
L¹ is selected from the group consisting of oxygen (-O-), -R⁴-C(=O)-O-, and -O-R^{4a}-O-C(=O)- ; wherein R⁴ is selected from the group consisting of C₁-C₃₀ alkylene, C₂-C₃₀ alkenylene, C₂-C₃₀ alkynylene, C₁-C₃₀ heteroalkylene, C₂-C₃₀ heteroalkenylene, C₂-C₃₀ heteroalkynylene, C₆-C₃₀ arylene, C₇-C₃₀ alkylarylene, C₈-C₃₀ alkenylarylene, C₈-C₃₀ alkynylarylene, and C₂-C₃₀ heteroarylene; and R^{4a} is selected from the group consisting of C₁-C₃₀ alkylene, C₂-C₃₀ alkenylene, C₂-C₃₀ alkynylene, C₁-C₃₀ heteroalkylene, C₂-C₃₀ heteroalkenylene, C₂-C₃₀ heteroalkynylene, C₆-C₃₀ arylene, C₇-C₃₀ alkylarylene, C₈-C₃₀ alkenylarylene, C₈-C₃₀ alkynylarylene, and C₂-C₃₀ heteroarylene;
L⁴ is selected from the group consisting of a bond, and optionally substituted phenoxy (-C₆H₄-O-);
L⁵ is selected from the group consisting of a bond and -CH₂-;
Q² at each occurrence is independently hydrogen or halogen; or two adjacent Q² form a double bond when adjacent Qs are present; or Z² and an adjacent Q² form a double bond;
Z² is hydrogen, -N(R^{x})C(=O)R⁵, -N(R^{x})COOR⁶, -C(O)OR⁷ or -C(O)NR^{x}R^{y}, or Z² and an adjacent Q² form a double bond; and
wherein R⁵, R⁶, R⁷, R^{x} and R^{y} are independently at each occurrence selected from the group consisting of hydrogen, alkyl, aryl, alkylaryl, arylalkyl, heteroalkyl, and heteroalkylaryl, wherein the non-hydrogen groups contain up to 30 carbon atoms, wherein the heteroalkyl group contains from 1 to 10 heteroatoms independently selected from O, N and S, and the heteroalkylaryl group contains from 1 to 3 heteroatoms independently selected from O, N and S;
with the proviso that the diphenol compound is not

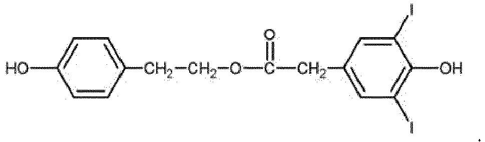

In another aspect, the present invention provides a polymer composition comprising a biocompatible polymer described herein.

In another aspect, the present invention provides a medical device comprising a biocompatible polymer described herein. In a preferred embodiment, the medical device is a stent.

These and other embodiments are described in greater detail below.

### DETAILED DESCRIPTION OF THE INVENTION

To meet the need of versatile moldable biodegradable and biocompatible polymers made using relatively nontoxic monomeric starting materials, the present application describes a variety of such monomers and polymers prepared from these monomers.

One aspect of the invention is directed to a biocompatible polymer comprising a recurring unit of Formula (XVIII): 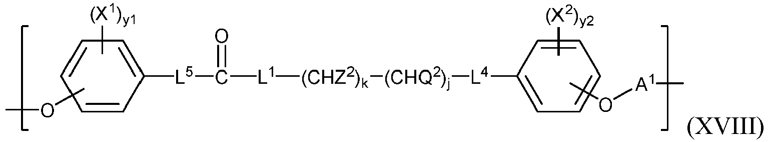 where:
j and k are each independently zero or an integer selected from 1 to 6, provided that j and k are not both 0;
X¹ and X² are each independently bromine (Br) or iodine (I), and y1 and y2 are each independently 0, 1, 2, 3, or 4;
L¹ is selected from the group consisting of oxygen (-O-), -R⁴-C(=O)-O-, and -O-R^{4a}-O-C(=O)- ; wherein R⁴ is selected from the group consisting of C₁-C₃₀ alkylene, C₂-C₃₀ alkenylene, C₂-C₃₀ alkynylene, C₁-C₃₀ heteroalkylene, C₂-C₃₀ heteroalkenylene, C₂-C₃₀ heteroalkynylene, C₆-C₃₀ arylene, C₇-C₃₀ alkylarylene, C₈-C₃₀ alkenylarylene, C₈-C₃₀ alkynylarylene, and C₂-C₃₀ heteroarylene; and R^{4a} is selected from the group consisting of C₁-C₃₀ alkylene, C₂-C₃₀ alkenylene, C₂-C₃₀ alkynylene, C₁-C₃₀ heteroalkylene, C₂-C₃₀ heteroalkenylene, C₂-C₃₀ heteroalkynylene, C₆-C₃₀ arylene, C₇-C₃₀ alkylarylene, C₈-C₃₀ alkenylarylene, C₈-C₃₀ alkynylarylene, and C₂-C₃₀ heteroarylene;
L⁴ is selected from the group consisting of a bond, and optionally substituted phenoxy (-C₆H₄-O-);
L⁵ is selected from the group consisting of a bond and -CH₂-;
Q² at each occurrence is independently hydrogen or halogen; or two adjacent Q² form a double bond when adjacent Qs are present; or Z² and an adjacent Q² form a double bond;
Z² is hydrogen, -N(R^{x})C(=O)R⁵, -N(R^{x})COOR⁶, -C(O)OR⁷ or -C(O)NR^{x}R^{y}, or Z² and an adjacent Q² form a double bond;
wherein R⁵, R⁶, R⁷, R^{x} and R^{y} are independently at each occurrence selected from the group consisting of hydrogen, alkyl, aryl, alkylaryl, arylalkyl, heteroalkyl, and heteroalkylaryl, wherein the non-hydrogen groups contain up to 30 carbon atoms, wherein the heteroalkyl group contains from 1 to 10 heteroatoms independently selected from O, N and S, and the heteroalkylaryl group contains from 1 to 3 heteroatoms independently selected from O, N and S; and
wherein A¹ is selected from the group consisting of: 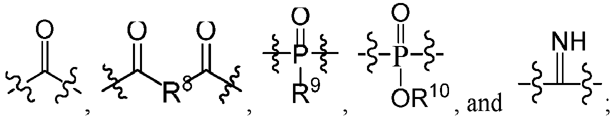
wherein R⁸ is selected from a bond, C₁-C₃₀ alkylene, C₂-C₃₀ alkenylene, C₂-C₃₀ alkynylene; C₁-C₃₀ heteroalkylene, C₂-C₃₀ heteroalkenylene, C₂-C₃₀ heteroalkynylene, C₇-C₃₀ heteroalkylarylene, C₈-C₃₀ heteroalkenylarylene, C₈-C₃₀ heteroalkynylarylene, C₇-C₃₀ alkylarylene, C₈-C₃₀ alkenylarylene, C₈-C₃₀ alkynylarylene, and C₂-C₃₀ heteroarylene; and
wherein R⁹ and R¹⁰ are each independently selected from H, C₁-C₃₀ alkyl, C₁-C₃₀ heteroalkyl, C₂-C₃₀ alkenyl, C₂-C₃₀ alkynyl, C₂-C₃₀ heteroalkenyl, and C₂-C₃₀ heteroalkynyl;
with the proviso that the biocompatible polymer is not poly(4'-hydroxyphenyethyl-2-(4-hydroxy-3,5-diiodophenyl)acetate carbonate).

In one embodiment of the biocompatible polymer L¹ is oxygen and L⁴ is a bond, providing a recurring unit of Formula (XVIIIa):

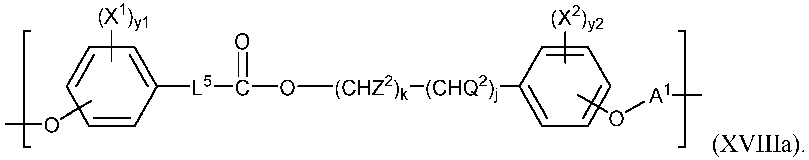

In one embodiment of the biocompatible polymer L¹ is oxygen and L⁴ is a bond, and Q2 is hydrogen, providing a recurring unit of Formula (XVIIIb):

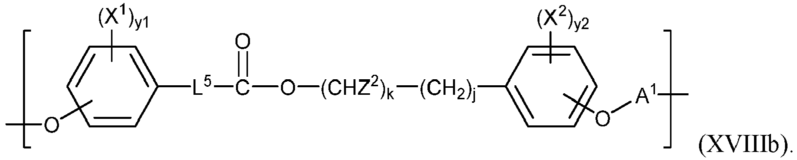

In one embodiment of the biocompatible polymer L⁵ is a bond, providing a recurring unit of Formula (XVIIIc):

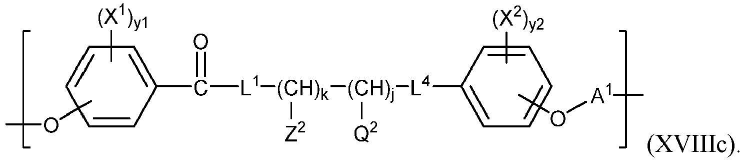

In one embodiment of the biocompatible polymer j = k = 1, L1 is oxygen, L4 and L⁵ are each a bond, and Q² and Z² are each hydrogen, providing a recurring unit of Formula (XVIIId):

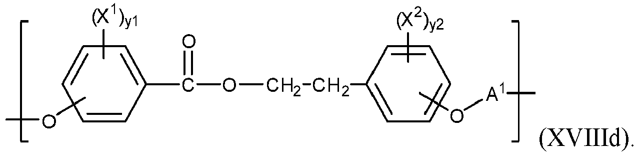

One embodiment of the biocompatible polymer comprising a recurring unit of Formula (XVIII) further comprises a tyrosol recurring unit and is characterized by the Formula (XVIIIe):

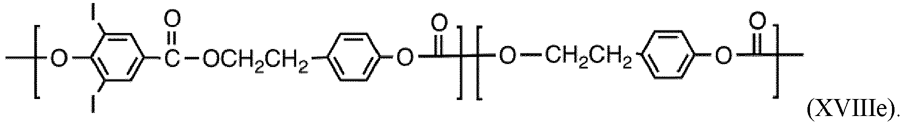

One embodiment of the biocompatible polymer comprising a recurring unit of Formula (XVIII) further comprises a p-hydroxybenzoyl tyrosol recurring unit and is characterized by the Formula (XVIIIf):

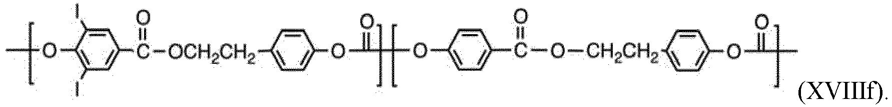

One embodiment of the biocompatible polymer comprising a recurring unit of Formula (XVIII) further comprises an α-hydroxyacid recurring unit and is characterized by the Formula (XVIIIg): 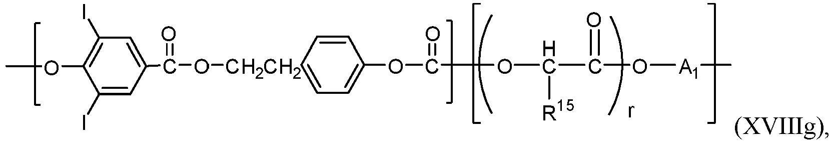 where R¹⁵ = H or CH₃, and r = 1 to 300.

One embodiment of the biocompatible polymer comprising a recurring unit of Formula (XVIII) further comprises an iodinated tyrosol recurring unit and is characterized by the Formula (XVIIIh):

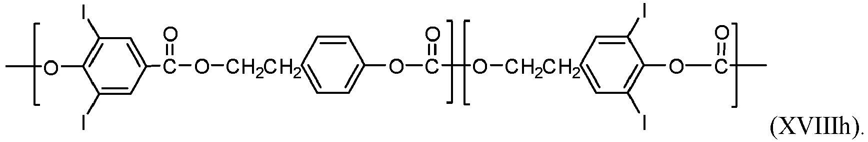

A related aspect of the invention is directed to a biocompatible polymer comprising a recurring unit of Formula (XIX): 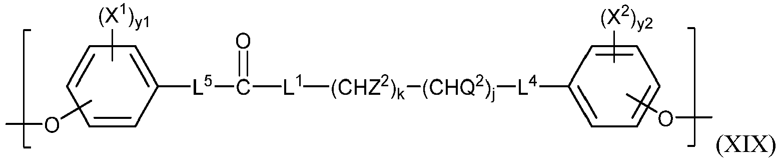 where
j and k are each independently zero or an integer selected from 1 to 6, provided that j and k are not both 0;
X¹ and X² are each independently bromine (Br) or iodine (I), and y1 and y2 are each independently 0, 1, 2, 3, or 4;
L¹ is selected from the group consisting of oxygen (-O-), -R⁴-C(=O)-O-, and -O-R^{4a}-O-C(=O)-; wherein ^{R4} is selected from the group consisting of C₁-C₃₀ alkylene, C₂-C₃₀ alkenylene, C₂-C₃₀ alkynylene, C₁-C₃₀ heteroalkylene, C₂-C₃₀ heteroalkenylene, C₂-C₃₀ heteroalkynylene, C₆-C₃₀ arylene, C₇-C₃₀ alkylarylene, C₈-C₃₀ alkenylarylene, C₈-C₃₀ alkynylarylene, and C₂-C₃₀ heteroarylene; and R^{4a} is selected from the group consisting of C₁-C₃₀ alkylene, C₂-C₃₀ alkenylene, C₂-C₃₀ alkynylene, C₁-C₃₀ heteroalkylene, C₂-C₃₀ heteroalkenylene, C₂-C₃₀ heteroalkynylene, C₆-C₃₀ arylene, C₇-C₃₀ alkylarylene, C₈-C₃₀ alkenylarylene, C₈-C₃₀ alkynylarylene, and C₂-C₃₀ heteroarylene;
L⁴ is selected from a bond, and optionally substituted phenoxy (-C₆H₄-O-);
L⁵ is selected from a bond and -CH₂-;
Q² at each occurrence is independently hydrogen or halogen; or two adjacent Q² form a double bond when adjacent Qs are present; or Z² and an adjacent Q² form a double bond;
Z² is hydrogen, -N(R^{x})C(=O)R⁵, -N(R^{x})COOR⁶, -C(O)OR⁷ or -C(O)NR^{x}R^{y}, or Z² and an adjacent Q² form a double bond; and
wherein R⁵, R⁶, R⁷, R^{x} and R^{y} are independently at each occurrence selected from the group consisting of hydrogen, alkyl, aryl, alkylaryl, arylalkyl, heteroalkyl, and heteroalkylaryl, wherein the non-hydrogen groups contain up to 30 carbon atoms, wherein the heteroalkyl group contains from 1 to 10 heteroatoms independently selected from O, N and S, and the heteroalkylaryl group contains from 1 to 3 heteroatoms independently selected from O, N and S.

In one embodiment of the biocompatible polymer L¹ is oxygen and L⁴ is a bond, providing a recurring unit of Formula (XIXa):

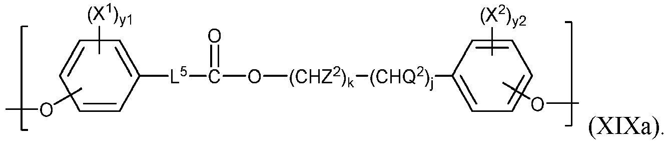

In one embodiment of the biocompatible polymer L¹ is oxygen and L⁴ is a bond, and Q² is hydrogen, providing a recurring unit of Formula (XIXb):

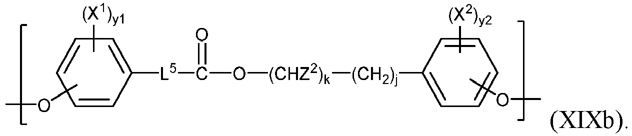

In one embodiment of the biocompatible polymer L⁵ is a bond, providing a recurring unit of Formula (XIXc):

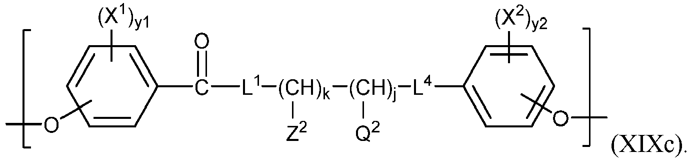

In one embodiment of the biocompatible polymer j = k = 1, L¹ is oxygen, L⁴ and L⁵ are each a bond, and Q² and Z² are each hydrogen, providing a recurring unit of Formula (XIXd):

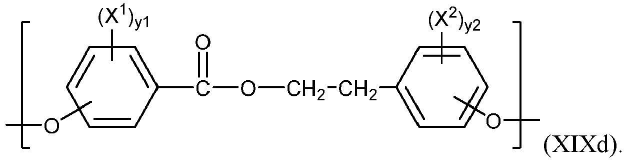

In some embodiments of the above polymers which comprise recurring units of Formulae (XVIII), (XVIIIa-h), (XIX) and (XIXa-d) the phenyl groups of the recurring unit have the phenolic oxygens in the para-position. In some embodiments of the polymers comprising recurring units of Formulae (XVIII), (XVIIIa-h), (XIX) and (XIXa-d) L⁴ is a bond, L¹ is oxygen, and Q² and Z² are both hydrogen. In some embodiments of the polymers comprising recurring units of Formulae (XVIII), (XVIIIa-h), (XIX) and (XIXa-d) X¹ and X² are iodine, and at least one of y1 and y2 is non-zero. In some embodiments of the polymers comprising recurring units of Formulae (XVIII), (XVIIIa-h), (XIX) and (XIXa-d) X¹ and X² are iodine, at least one of y1 and y2 is 2, and the phenyl groups of the recurring unit have the phenolic oxygens in the para-position.

In some embodiments of the polymers comprising recurring units of Formulae (XVIII), (XVIIIa-h), (XIX) and (XIXa-d), the polymer is a polycarbonate and further comprises a recurring unit of optionally iodinated tyrosol.

In some embodiments, any of the above-described biocompatible copolymers further comprise a tyrosol recurring unit of the formula: 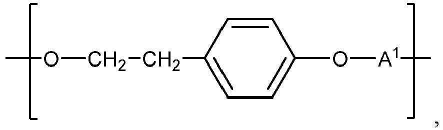
wherein A¹ is selected from the group consisting of: 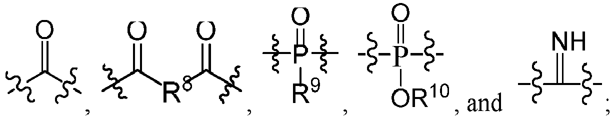
wherein R⁸ is selected from a bond, C₁-C₃₀ alkylene, C₂-C₃₀ alkenylene, C₂-C₃₀ alkynylene; C₁-C₃₀ heteroalkylene, C₂-C₃₀ heteroalkenylene, C₂-C₃₀ heteroalkynylene, C₇-C₃₀ heteroalkylarylene, C₈-C₃₀ heteroalkenylarylene, C₈-C₃₀ heteroalkynylarylene, C₇-C₃₀ alkylarylene, C₈-C₃₀ alkenylarylene, C₈-C₃₀ alkynylarylene, and C₂-C₃₀ heteroarylene; and wherein R⁹ and R¹⁰ are each independently selected from H, C₁-C₃₀ alkyl, C₁-C₃₀ heteroalkyl, C₂-C₃₀ alkenyl, C₂-C₃₀ alkynyl, C₂-C₃₀ heteroalkenyl, and C₂-C₃₀ heteroalkynyl.In some embodiments, any of the above-described biocompatible copolymers further comprise a recurring unit of the formula: 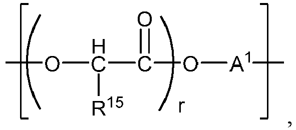
wherein R¹⁵ is H or CH₃, r is 1 to 300, and A¹ is as described above.

In some embodiments, any of the above-described biocompatible copolymers is characterized as being a block copolymer with a macromer of the formula: 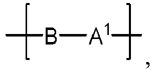
wherein B is -O-((CH₂)ₚ-O)_{q}- or -O-(CH₂CH(CH₃)-O)_{q}-;
p is an integer ranging from 1 to 6, and q is 5 to 3000; and
A¹ is as described above.

In some embodiments, any of the above-described biocompatible copolymers further comprise a p-hydroxybenzoate recurring unit of the formula: 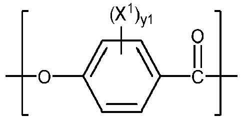 wherein X¹ is selected from Br and I and y1 = 0, 1 or 2.

Another aspect of the invention is directed to a biocompatible polyurethane comprising an optionally iodinated tyrosine or an optionally iodinated tyrosine ester recurring unit, and a second recurring unit selected from the group consisting of HBA (hydroxybenzoid acid), mono-iodinated HBA and di-iodinated HBA.

Another aspect of the invention is directed to a diphenol monomer compound having the Formula (XX): 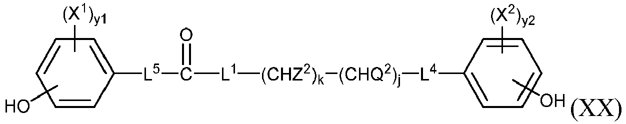 where:
j and k are each independently zero or an integer selected from 1 to 6, provided that j and k are not both 0;
X¹ and X² are each independently bromine (Br) or iodine (I), and y¹ and y² are each independently 0, 1, 2, 3, or 4;
L¹ is selected from oxygen (-O-), -R⁴-C(=O)-O-, and -O-R^{4a}-O-C(=O)- ; wherein R⁴ is selected from the group consisting of C₁-C₃₀ alkylene, C₂-C₃₀ alkenylene, C₂-C₃₀ alkynylene, C₁-C₃₀ heteroalkylene, C₂-C₃₀ heteroalkenylene, C₂-C₃₀ heteroalkynylene, C₆-C₃₀ arylene, C₇-C₃₀ alkylarylene, C₈-C₃₀ alkenylarylene, C₈-C₃₀ alkynylarylene, and C₂-C₃₀ heteroarylene; and R^{4a} is selected from C₁-C₃₀ alkylene, C₂-C₃₀ alkenylene, C₂-C₃₀ alkynylene, C₁-C₃₀ heteroalkylene, C₂-C₃₀ heteroalkenylene, C₂-C₃₀ heteroalkynylene, C₆-C₃₀ arylene, C₇-C₃₀ alkylarylene, C₈-C₃₀ alkenylarylene, C₈-C₃₀ alkynylarylene, and C₂-C₃₀ heteroarylene;
L⁴ is selected from a bond, and optionally substituted phenoxy (-C₆H₄-O-);
L⁵ is selected from a bond and -CH₂-;
Q² at each occurrence is independently hydrogen or halogen; or two adjacent Q² form a double bond when adjacent Qs are present; or Z² and an adjacent Q² form a double bond;
Z² is hydrogen, -N(R^{x})C(=O)R⁵, -N(R^{x})COOR⁶, -C(O)OR⁷ or -C(O)NR^{x}R^{y}, or Z² and an adjacent Q² form a double bond; and
wherein R⁵, R⁶, R⁷, R^{x} and R^{y} are independently at each occurrence selected from the group consisting of hydrogen, alkyl, aryl, alkylaryl, arylalkyl, heteroalkyl, and heteroalkylaryl, wherein the non-hydrogen groups contain up to 30 carbon atoms, wherein the heteroalkyl group contains from 1 to 10 heteroatoms independently selected from O, N and S, and the heteroalkylaryl group contains from 1 to 3 heteroatoms independently selected from O, N and S;
with the proviso that the diphenol compound is not

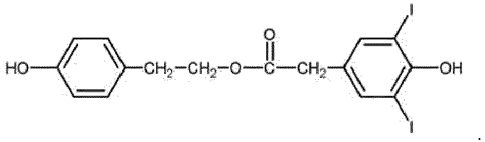

In one embodiment of the diphenol compound the phenolic hydroxy groups are in the para-position, providing a diphenol compound having the Formula (XXa):

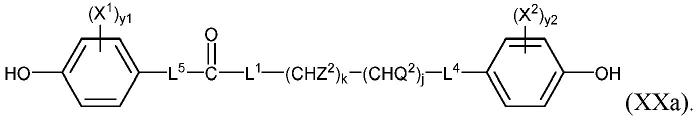

In one embodiment of the diphenol compound L⁵ is a bond. In one embodiment of the diphenol compound L⁴ and L⁵ are each a bond, L¹ is oxygen, Z² is hydrogen and Q² is hydrogen. In one embodiment of the diphenol compound L⁴ and L⁵ are each a bond, L¹ is oxygen, Z² is hydrogen, Q² is hydrogen and k = j = 1. In one embodiment of the diphenol compound L⁴ and L⁵ are each a bond, L¹ is oxygen, Q² and Z² are both hydrogen, k = j = 1, X¹ and X² are iodine, and at least one of y1 and y2 is non-zero.

Polymer compositions can be prepared comprising any of the above-described the biocompatible polymers and at least one pharmaceutically acceptable carrier, additive or excipient. In some embodiments the polymer compositions comprise polymers containing one or more of the recurring units of Formulae (XVIII), (XVIIIa-h), (XIX) and (XIXa-d).

Medical devices can be fabricated from any of the above-described biocompatible polymers. In some embodiments the medical device comprises a biocompatible polymer containing one or more of the recurring units of Formulae (XVIII), (XVIIIa-h), (XIX) and (XIXa-d). The medical device can further comprise a biologically active compound incorporated into the biocompatible polymer. In some embodiments the biologically active compound can be selected from the group consisting of chemotherapeutic agents, non-steroidal anti-inflammatory agents, steroidal anti-inflammatory agents, and wound healing agents. In certain embodiments the medical device is a stent. Preferably the stent comprises any one or more of the biocompatible polymers described above; and particularly of one or more of the biocompatible polymers comprising one or more of the recurring units of Formulae (XVIII), (XVIIIa-h), (XIX) and (XIXa-d).

### Monomer and Polymer Syntheses

The polymers described herein (including, e.g, polymers comprising a recurring unit selected from the group of recurring units represented by Formula (XVIII), Formulae (XVIIIa-h), Formula (XIX) and Formulae (XIXa-d) may be synthesized by various conventional reactions known in the art.

For example, the diphenolic monomer compounds can be reacted with aliphatic or aromatic dicarboxylic acids in a carbodiimide-mediated direct polyesterification using DPTS as a catalyst to form aliphatic or aromatic polyarylates. Examples of dicarboxylic acids suitable for polymerization to form polyarylates have a structure of Formula (XVII): 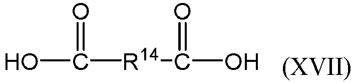 in which, for the aliphatic polyarylates, R¹⁴ is selected from saturated and unsaturated, substituted and unsubstituted alkylene or alkylarylene groups containing up to 18 carbon atoms, and preferably from 2 to 12 carbon atoms. For the aromatic polyarylates, R¹⁴ is selected from arylene groups containing up to 18 carbon atoms and preferably from 6 to 12 carbon atoms. In some embodiments, R¹⁴ is defined as above for R⁸.

R¹⁴ is preferably selected so that the dicarboxylic acids employed as starting materials are either important naturally-occurring metabolites or highly biocompatible compounds. Examples of preferred aliphatic dicarboxylic acid starting materials are described elsewherein herein.

The polyarylates can also be prepared by the method disclosed by Higashi et al., J. Polym. Sci.: Polym. Chem. Ed., 21, 3233-9 (1983) using arylsulfonyl chloride as the condensing agent, by the process of Higashi et al., J. Polym. Sci.: Polym. Chem. Ed., 21, 3241-7 (1983) using diphenyl chlorophosphate as the condensing agent, by the process of Higashi et al., J. Polym. Sci.: Polym. Chem. Ed., 24, 97-102 (1986) using thionyl chloride with pyridine as the condensing agent, or by the process of Elias, et al., Makromol. Chem., 182, 681-6 (1981) using thionyl chloride with triethylamine. A preferred polyesterification procedure is the method disclosed by Moore et al., Macromol., 23, 65-70 (1990) utilizing carbodiimide coupling reagents as the condensing agents with the specially designed catalyst DPTS.

A particularly preferred polyesterification technique modifies the method of Moore to utilize an excess of the carbodiimide coupling reagent. This technique tends to produce aliphatic polyarylates having molecular weights greater than those obtained by Moore. Essentially any carbodiimide commonly used as a coupling reagent in peptide chemistry can be used as a condensing agent in the preferred polyesterification process. Such carbodiimides are well-known and disclosed in Bodanszky, Practice of Peptide Synthesis (Springer-Verlag, New York, 1984) and include dicyclohexylcarbodiimide, diisopropylcarbodiimide, 1-(3-dimethylaminopropyl)-3-ethyl carbodiimide hydrochloride, N-cyclohexyl-N'-(2'-morpholinoethyl)carbodiimide-metho-p-toluene sulfonate, N-benzyl-N'-3'- dimethyl-aminopropyl-carbodiimide hydrochloride, 1-ethyl-3-(3-dimethylaminopropyl)-carbodiimide methiodide, N-ethylcarbodiimide hydrochloride, and the like. The preferred carbodiimides are dicyclohexyl carbodiimide and diisopropylcarbodiimide.

An esterification reaction mixture can generally be formed by contacting exactly equimolar quantities of the diphenol and the dicarboxylic acid in a solvent for the diphenol and the dicarboxylic acid. Suitable solvents include methylene chloride, tetrahydrofuran, dimethylformamide, chloroform, carbon tetrachloride and N-methyl pyrrolidinone. It is not necessary to bring all reagents into complete solution prior to initiating the polyesterification reaction, although the polymerization of slightly soluble monomers such as desaminotyrosyltyrosine ethyl ester and succinic acid will typically yield higher molecular weight polymers when the amount of solvent is increased. The reaction mixture can also be heated gently to aid in the partial dissolution of the reactants.

The polyarylates can be worked up and isolated by known methods commonly employed in the field of synthetic polymers to produce a variety of useful articles with valuable physical and chemical properties, all derived from tissue compatible monomers. The useful articles can be shaped by conventional polymer-forming techniques such as extrusion, compression molding, injection molding, and the like. Molded articles prepared from the polyarylates are useful, inter alia, as degradable biomaterials for medical implant applications. Such applications include the use of the molded articles as vascular grafts and stents, bone plates, sutures, implantable sensors, barriers for surgical adhesion prevention, implantable drug delivery devices and other therapeutic aids and articles which decompose harmlessly within a known period of time.

### Diphenolic ester monomers comprising halogenated HBA.

Scheme 1a shows the production of a non-halogenated diphenolic ester monomer by reaction of 4-Hydroxybenzoic Acid with tyrosol. Methods used may include, e.g., heating 4-hydroxybenzoic acid (HBA) together with tyrosol in 4-toluenesulfonic acid monohydrate, and 1,2-dichloroethane (DCE) under reflux. Alternative method may include Fisher synthesis.and heating with catalytic amount of sulfuric acid with toluene under reflux. The product is the tyrosol ester of 4-hydroxybenzoic acid, or tyrosyl(4-hydroxybenzoate), abbreviated herein as BTy.

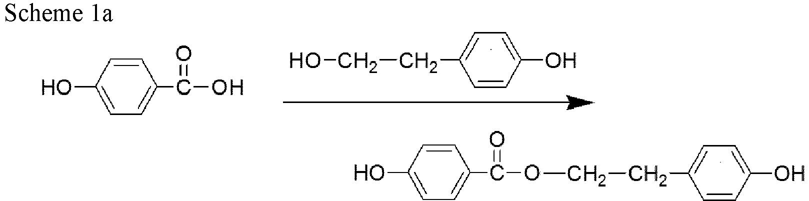

Scheme 1b shows the production of halogenated enated diphenolic ester monomer by reaction of tyrosyl(4-hydroxybenzoate) to iodinate the constituent rings of the diphenol, e.g., the reaction may be carried out in ethanol using iodine in the presence of sulfuric acid, wherein aqueous H₂O₂ or potassium iodate is used as the oxidizing agent. The product is 3,5-diiodotyrosyl(4-hydroxy-3,5-diiodophenyl)benzoate.

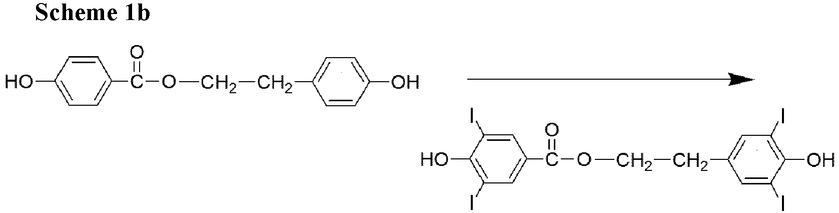

Polyiminocarbonates are structurally related to polycarbonates. The polyiminocarbonates have imino groups in the places typically occupied by carbonyl oxygen in the polycarbonates. Thus, the polyiminocarbonates have linkages according to the formula:

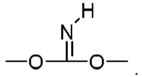

Inclusion of iminocarbonate linkages may impart a significant degree of hydrolytic instability to the polymer. The polyiminocarbonates have desirable mechanical properties akin to those of the corresponding polycarbonates.

Starting materials described herein are available commercially, are known, or may be prepared by methods known in the art. Additionally, starting materials not described herein are available commercially, are known, or may be prepared by methods known in the art.

Starting materials may have the appropriate substituents to ultimately give desired products with the corresponding substituents. Alternatively, substituents may be added at any point of synthesis to ultimately give desired products with the corresponding substituents.

The synthetic schemes illustrated herein show methods that may be used to prepare the compounds of preferred embodiments. One skilled in the art will appreciate that a number of different synthetic reaction schemes may be used to synthesize the compounds of preferred embodiments. Further, one skilled in the art will understand that a number of different solvents, coupling agents and reaction conditions may be used in the synthetic reactions to yield comparable results.

One skilled in the art will appreciate variations in the sequence and, further, will recognize variations in the appropriate reaction conditions from the analogous reactions shown or otherwise known which may be appropriately used in the processes above to make the compounds of preferred embodiments.

In the processes described herein for the preparation of the compounds of preferred embodiments, the requirements for protective groups are generally well recognized by one skilled in the art of organic chemistry, and accordingly the use of appropriate protecting groups is necessarily implied by the processes of the schemes herein, although such groups may not be expressly illustrated. Introduction and removal of such suitable protecting groups are well known in the art of organic chemistry; see for example, T. W. Greene, "Protective Groups in Organic Synthesis", Wiley (New York), 1999.

The products of the reactions described herein can be isolated by conventional means such as extraction, distillation, chromatography, and the like.

The salts of the compounds described herein can be prepared by reacting the appropriate base or acid with a stoichiometric equivalent of the compound.

In some embodiments, the polymer comprises poly(ether carbonate) wtih tyrosolbioactive moiety. A desaminotyrosyl-tyrosine dipeptide can be combined with the PEG in methylene chloride and phosgene can be added as a solution in toluene. The reaction would be completed in around 9 minutes. In some embodiments, this reaction is carried out for from 1-60 minutes. In an embodiment, the polymer comprises poly(tyrosine carbonate) pendant bioactive moiety groups. In some embodiments, the polymer comprises poly(ether carbonate) tyrosine-diol copolymer with a bioactive moiety in the backbone. In some embodiments, the polymer comprises poly(ether carbonate) tyrosine-diol copolymer with a pendant bioactive moiety. In some embodiments, the polymer comprises poly(ether ester) tyrosine-bioactive moiety-diacid copolymer. In some embodiments, the polymer comprises poly(imino carbonate) tyrosine-bioactive moiety-copoymer. In some embodiments, the polymer comprises poly(imono tyrosine) with pendant PEG groups.

In another aspect the present invention provides a medical device that comprises a polymer and/or polymer composition as described herein. For example, an embodiment provides a stent that comprises a polymer composition as described herein.

### DEFINITIONS

The term "biodegradable," as used herein, refers to a property of polymer whose molecular weight goes down because of hydrolysis or enzymatic reactions under physiological conditions such that the polymer is transformed into lower molecular weight oligomers in a period not to exceed four (4) years.

The term "oligomer," as used herein, refers to a hydrolyzed product of a polymer, whose molecular weight is less than 10% of the original polymer.

The terms "alkyl", "alkylene" and similar terms have the usual meaning known to those skilled in the art and thus may be used to refer to straight or branched hydrocarbon chain fully saturated (no double or triple bonds) hydrocarbon group. Terminal alkyl groups, e.g., of the general formula -CₙH₂ₙ₊₁, may be referred to herein as "alkyl" groups, whereas linking alkyl groups, e.g., of the general formula -(CH₂)ₙ-, may be referred to herein as "alkylene" groups. The alkyl group may have 1 to 50 carbon atoms (whenever it appears herein, a numerical range such as "1 to 50" refers to each integer in the given range; *e.g.,* "1 to 50 carbon atoms" means that the alkyl group may consist of 1 carbon atom, 2 carbon atoms, 3 carbon atoms, *etc*., up to and including 50 carbon atoms, although the present definition also covers the occurrence of the term "alkyl" where no numerical range is designated). The alkyl group may also be a medium size alkyl having 1 to 30 carbon atoms. The alkyl group could also be a lower alkyl having 1 to 5 carbon atoms. The alkyl group of the compounds may be designated as "C₁-C₄ alkyl" or similar designations. By way of example only, "C₁-C₄ alkyl" indicates that there are one to four carbon atoms in the alkyl chain, i.e., the alkyl chain is selected from the group consisting of methyl, ethyl, propyl, isopropyl, n-butyl, iso-butyl, sec-butyl, and t-butyl. Typical alkyl groups include, but are in no way limited to, methyl, ethyl, propyl, isopropyl, butyl, isobutyl, tertiary butyl, pentyl, hexyl and the like.

The alkyl group may be substituted or unsubstituted. When substituted, the substituent group(s) is(are) one or more group(s) individually and independently selected from alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, protected hydroxyl, alkoxy, aryloxy, acyl, ester, mercapto, alkylthio, arylthio, cyano, halogen, carbonyl, thiocarbonyl, O-carbamyl, N-carbamyl, O-thiocarbamyl, N-thiocarbamyl, C-amido, N-amido, S-sulfonamido, N-sulfonamido, C-carboxy, protected C-carboxy, O-carboxy, isocyanato, thiocyanato, isothiocyanato, nitro, silyl, sulfenyl, sulfinyl, sulfonyl, haloalkyl, haloalkoxy, trihalomethanesulfonyl, trihalomethanesulfonamido, and amino, including mono- and di-substituted amino groups, and the protected derivatives thereof. Wherever a substituent is described as being "optionally substituted" that substitutent may be substituted with one of the above substituents.

An "aralkyl" is an aryl group connected, as a substituent, via an alkylene group. The alkylene and aryl group of an aralkyl may be substituted or unsubstituted. Examples include but are not limited to benzyl, substituted benzyl, 2-phenylethyl, 3-phenylpropyl, and naphtylalkyl. In some cases, the alkylene group is a lower alkylene group. An alkylaryl group may be substituted or unstubstituted.

As noted above, alkyl groups may link together other groups, and in that context may be referred to as alkylene groups. Alkylene groups are thus biradical tethering groups, forming bonds to connect molecular fragments via their terminal carbon atoms. Examples include but are not limited to methylene (-CH₂-), ethylene (-CH₂CH₂-), propylene (-CH₂CH₂CH₂-), and butylene (-(CH₂)₄-) groups. An alkylene group may be substituted or unsubstituted.

The terms "alkenyl", "alkenylene" and similar terms have the usual meaning known to those skilled in the art and thus may be used to refer to an alkyl or alkylene group that contains in the straight or branched hydrocarbon chain containing one or more double bonds. An alkenyl group may be unsubstituted or substituted. When substituted, the substituent(s) may be selected from the same groups disclosed above with regard to alkyl group substitution unless otherwise indicated.

An "amide" is a chemical moiety with formula -(R)ₙ-C(O)NHR' or -(R)ₙ-NHC(O)R', where R and R' are independently selected from the group consisting of alkyl, cycloalkyl, aryl, heteroaryl (bonded through a ring carbon) and heteroalicyclic (bonded through a ring carbon), and where n is 0 or 1. An amide may be an amino acid or a peptide molecule attached to a molecule of the present invention, thereby forming a prodrug. An "amide linkage" is an amide group (-C(O)NH-) that links two chemical moieties to one another.

Any amine, hydroxy, or carboxyl side chain on the compounds disclosed herein can be esterified or amidified. The procedures and specific groups to be used to achieve this end are known to those of skill in the art and can readily be found in reference sources such as Greene and Wuts, Protective Groups in Organic Synthesis, 3rd Ed., John Wiley & Sons, New York, N.Y., 1999.

As used herein, "aryl" refers to a carbocyclic (all carbon) ring or two or more fused rings (rings that share two adjacent carbon atoms) that have a fully delocalized pi-electron system. Examples of aryl groups include, but are not limited to, benzene, naphthalene and azulene. An aryl group may be substituted or unsubstituted. When substituted, hydrogen atoms are replaced by substituent group(s) that is(are) one or more group(s) independently selected from alkyl, alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, protected hydroxyl, alkoxy, aryloxy, acyl, ester, mercapto, alkylthio, arylthio, cyano, halogen, carbonyl, thiocarbonyl, O-carbamyl, N-carbamyl, O-thiocarbamyl, N-thiocarbamyl, C-amido, N-amido, S-sulfonamido, N-sulfonamido, C-carboxy, protected C-carboxy, O-carboxy, isocyanato, thiocyanato, isothiocyanato, nitro, silyl, sulfenyl, sulfinyl, sulfonyl, haloalkyl, haloalkoxy, trihalomethanesulfonyl, trihalo-methanesulfonamido, and amino, including mono- and di-substituted amino groups, and the protected derivatives thereof. When substituted, substituents on an aryl group may form a non-aromatic ring fused to the aryl group, including a cycloalkyl, cycloalkenyl, cycloalkynyl, and heterocyclyl.

As used herein, "heteroalkyl" refers to an alkyl group where one or more carbon atoms has been replaced with a heteroatom, that is, an element other than carbon, including but not limited to, nitrogen, oxygen and sulfur.

The terms "heteroalkyl", "heteroalkylene," and similar terms have the usual meaning known to those skilled in the art and thus may be used to refer to an alkyl group or alkylene group as described herein in which one or more of the carbons atoms in the backbone of alkyl group or alkylene group has been replaced by a heteroatom such as nitrogen, sulfur and/or oxygen. Likewise, the term "heteroalkenylene" may be used to refer to an alkenyl or alkenylene group in which one or more of the carbons atoms in the backbone of alkyl group or alkylene group has been replaced by a heteroatom such as nitrogen, sulfur and/or oxygen.

As used herein, "heteroaryl" refers to an aryl group where one or more carbon atoms has been replaced with a heteroatom, that is, an element other than carbon, including but not limited to, nitrogen, oxygen and sulfur.

For convenience and conciseness, sometimes the terms "alkyl", "alkenyl", "alkynyl", "aryl", "heteroaryl", and "alkylaryl", or the like, may be used to refer to the corresponding linking groups when they serve to connect two moieties of a molecule, either monomeric or polymeric, which is readily understood by those skilled in the art. That is, on such occasions "alkyl" should be interpreted as "alkylene"; "alkenyl" should be interpreted as "alkenylene"; "aryl" should be interpreted as "arylene"; and so on.

A "heavy atom" is an atom that, when attached to a polymer, renders the polymer easier to detect by an imaging technique as compared to a polymer that does not contain the heavy atom. Since many polymers contain relatively low atomic number atoms such as hydrogen, carbon, nitrogen, oxygen, silicon and sulfur, in most cases heavy atoms have an atomic number of 17 or greater. Preferred heavy atoms have an atomic number of 35 or greater and include bromine, iodine, bismuth, gold, platinum tantalum, tungsten and barium.

A "hydrocarbon" is an organic compound consisting entirely of hydrogen and carbon. Examples of hydrocarbons include unsubstituted alkyl groups, unsubstituted aryl groups, and unsubstituted alkylaryl groups. Any substitution to an alkyl group, aryl group, or alkylaryl group in a hydrocarbon would only comprise carbon and/or hydrogen atoms.

As used herein, the terms "macromer", "macromeric" and similar terms have the usual meaning known to those skilled in the art and thus may be used to refer to oligomeric and polymeric materials that are functionalized with end groups that are selected so that the macromers can be copolymerized with other macromers or monomers. A wide variety of macromers and methods for making them are known to those skilled in the art. Examples of suitable macromers include hydroxy endcapped polylactic acid macromers, hydroxy endcapped polyglycolic acid macromers, hydroxy endcapped poly(lactic acid-co-glycolic acid) macromers, hydroxy endcapped polycaprolactone macromers, poly(alkylene diol) macromers, hydroxy end-capped poly(alkylene oxide) macromers and hydroxy endcapped polydioxanone macromers.

As used herein, the terms "polymer", "polymeric" and similar terms have the usual meaning known to those skilled in the art and thus may be used to refer to homopolymers, copolymers (e.g., random copolymer, alternating copolymer, block copolymer, graft copolymer) and mixtures thereof. The repeating structural units of polymers may also be referred to herein as recurring units.

As used herein, the term "molecular weight" has the usual meaning known to those skilled in the art and thus reference herein to a polymer having a particular molecular weight will be understood as a reference to a polymer molecular weight in units of Daltons. Various techniques known to those skilled in the art, such as end group analysis (e.g., by ¹H NMR) and high pressure size exclusion chromatography (HPSEC, also known as gel permeation chromatography, "GPC"), may be used to determine polymer molecular weights. In some cases the molecular weights of polymers are further described herein using the terms "number average" molecular weight (Mn) and/or "weight average" molecular weight (Mw), both of which terms are likewise expressed in units of Daltons and have the usual meaning known to those skilled in the art.

Unless otherwise indicated, when a substituent is deemed to be "optionally substituted," it is meant that the substitutent is a group that may be substituted with one or more group(s) individually and independently selected from alkyl, alkenyl, alkylyl, cycloalkyl, aryl, heteroaryl, heteroalicyclic, hydroxyl, alkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, S-sulfonamido, N-sulfonamido, C-carboxy, O-carboxy, isocyanato, thiocyanato, isothiocyanato, nitro, silyl, trihalomethane-sulfonyl, and amino, including mono- and di-substituted amino groups, and the protected derivatives thereof. The protecting groups that may form the protective derivatives of the above subsituents are known to those of skill in the art and may be found in references such as Greene and Wuts, above.

The terms "radiopaque", "radio-opaque", "radiopacity", "radio-opacity", "radiopacifying" and similar terms have the usual meaning known to those skilled in the art and thus may be used to refer to polymer compositions that have been rendered easier to detect using medical imaging techniques (e.g., by X-ray and/or during fluoroscopy) being the incorporation of heavy atoms into the polymer composition. Such incorporation may be by mixing, e.g., by mixing an effective amount of a radiopacifying additive such as barium salt or complex, and/or by attachment of effective amounts of heavy atoms to one or more of the polymers in the polymer composition.

In certain configurations, polymer compositions may be inherently radiopaque. The term "inherently radiopaque" is used herein to refer to a polymer to which a sufficient number of heavy atoms are attached by covalent or ionic bonds to render the polymer radiopaque. This meaning is consistent with the understanding of those skilled in the art, see, e.g., U.S. Patent Publication No. 2006/0024266.

Whenever a group is described as being "optionally substituted" that group may be unsubstituted or substituted with one or more of the indicated substituents. Likewise, when a group is described as being "unsubstituted or substituted" if substituted, the substituent may be selected from one or more of the indicated substituents.

Unless otherwise indicated, when a substituent is deemed to be "optionally substituted," or "substituted" it is meant that the substituent is a group that may be substituted with one or more group(s) individually and independently selected from alkyl, alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, protected hydroxy, alkoxy, aryloxy, acyl, ester, mercapto, cyano, halogen, carbonyl, thiocarbonyl, O-carbamyl, N-carbamyl, O-thiocarbamyl, N-thiocarbamyl, C-amido, N-amido, S-sulfonamido, N-sulfonamido, C-carboxy, protected C-carboxy, O-carboxy, isocyanato, thiocyanato, isothiocyanato, nitro, silyl, sulfenyl, sulfinyl, sulfonyl, haloalkyl, haloalkoxy, trihalomethanesulfonyl, trihalomethanesulfonamido, and amino, including mono- and di-substituted amino groups, and the protected derivatives thereof. Similarly, the term "optionally ring-halogenated" may be used to refer to a group that optionally contains one or more (e.g., one, two, three or four) halogen substituents on the aryl and/or heteroaryl ring. The protecting groups that may form the protective derivatives of the above substituents are known to those of skill in the art and may be found in references such as Greene and Wuts, Protective Groups in Organic Synthesis, 3rd Ed., John Wiley & Sons, New York, NY, 1999.

It is understood that, in any compound described herein having one or more chiral centers, if an absolute stereochemistry is not expressly indicated, then each center may independently be of R-configuration or S-configuration or a mixture thereof. Thus, the compounds provided herein may be enantiomerically pure or be stereoisomeric mixtures. In addition it is understood that, in any compound having one or more double bond(s) generating geometrical isomers that can be defined as E or Z each double bond may independently be E or Z a mixture thereof. Likewise, all tautomeric forms are also intended to be included.

The following abbreviations are used to identify various iodinated compounds. TE stands for tyrosine ethyl ester, DAT stands for desaminotyrosine and DTE for desaminotyrosyl tyrosine ethyl ester. PTE stands for hydroxy-phenoxy-1-oxoethyl tyrosine ethyl ester. Ty stands for tyrosol. The polymer obtained by phosgenation of DTE is denoted as poly(DTE carbonate). An "I" before the abbreviation shows mono-iodination (e.g. ITE stands for mono-iodinated TE) and an I₂ before the abbreviation shows di-iodination (e.g. I₂DAT stands for di-iodinated DAT). In DTE, if the "I" is before D, it means the iodine is on DAT and if "I" is after D, it means the iodine is on the tyrosine ring (e.g. DI₂TE stands for DTE with 2 iodine atoms on the tyrosine ring). The following diagram illustrates this nomenclature further. 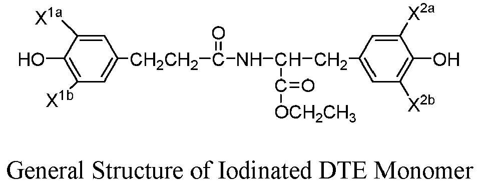
IDTE: X^{1a} = I, X^{1b} = H, X^{2a} = H, X^{2b} = H
I₂DTE: X^{1a} = I, X^{1b} = I, X^{2a} = H, X^{2b} = H
DI₂TE: X^{1a} = H, X^{1b} = H, X^{2a} = I, X^{2b} = I
IDITE: X^{1a} = I, X^{1b} = H, X^{2a} = I, X^{2b} = H

As used herein, in its most general form, the abbreviation "HBA" may refer to any or all of the hydroxy-arylcarboxylic acid moieties know as hydroxybenzoic acid, it being understood that there are three are different isomers of monohydroxybenzoic acid: 4-hydroxybenzoic acid (para-hydroxybenzoic acid, sometimes abbreviated PHBA), 3-hydroxybenzoic acid (meta-hydroxybenzoic acid), and 2-hydroxybenzoic acid (orthohydroxybenzoic acid or salicylic acid). It will be understood by one of ordinary skill in the art that any one of these may included in various ones of the general monomer and polymer concepts described herein with reference to hydroxybenzoic acid, being duly cognizant of any different chemical and biological properties or characteristics of each isomer. However, the most typically preferred examples of hydroxybenzoic acid herein include the 4-hydroxybenzoic acid isomer. For this reason, the terms hydroxybenzoic acid or HBA will herein generally refer to 4-hydroxybenzoic acid as a preferred example among the three possible isomers, unless otherwise specified.

For PTE, PTH, IPTE, I₂PTE, PI₂TE, etc., the DAT -CH₂CH₂- group is replaced with -OCH₂-.

As used herein, the abbreviations for any protective groups, amino acids and other compounds are, unless indicated otherwise, in accord with their common usage, recognized abbreviations, or the ILTPAC-ILTP Commission on Biochemical Nomenclature (See, Biochem. 11:942-944 (1972)).

The term "halogen atom," as used herein, means any one of the radio-stable atoms of column 7 of the Periodic Table of the Elements, e.g., fluorine, chlorine, bromine, or iodine, with bromine and iodine being preferred.

The term "ester" refers to a chemical moiety with formula -(R)ₙ-COOR', where R and R' are independently selected from the group consisting of alkyl, cycloalkyl, aryl, heteroaryl (bonded through a ring carbon) and heteroalicyclic (bonded through a ring carbon), and where n is 0 or 1. An "ester linkage" is an ester group that links two chemical moieties to one another.

The terms "purified," "substantially purified," and "isolated" as used herein refer to compounds disclosed herein being substantially free of other, dissimilar compounds with which the compounds of the invention are normally associated in their natural state, so that the compounds of the invention comprise at least 0.5%, 1%, 5%, 10%, or 20%, and most preferably at least 50% or 75% of the mass, by weight, of a given sample.

It is understood that the polymers described herein may be used in accordance with preferred aspects of the invention as a homogeneous polymer, as a copolymer, and/or as a polymer blend.

The bioresorbable, inherently radiopaque stents disclosed in accordance with preferred embodiments of the present invention may be used, for example, to temporarily treat a blood vessel as in traditional applications which generally include delivery through a catheter.

In some embodiments polymers prepared from sufficient amounts of the the monomeric starting materials described herein and having at least one bromine- or iodinesubstituted aromatic ring are radio-opaque, such as the polymers prepared from radiopaque diphenol compounds prepared according to the disclosure of U.S. Patent No. 6,475,477, as well as he disclosure of co-pending and commonly-owned U.S. Patent Application Serial No. 10/592,202. The iodinated and brominated diphenol monomers of the present invention can also be employed as radio-opacifying, biocompatible non-toxic additives for other polymeric biomaterials.

Bromine and iodine substituted aromatic monomers of the present invention can be prepared by well-known iodination and bromination techniques that can be readily employed by those of ordinary skill in the art in view of the guidance provided herein without undue experimentation. In some embodiments, the halogenated aromatic compounds from which the halogenated aromatic monomers of the present invention are prepared typically undergo ortho-directed halogenation. The term "ortho-directed" is used herein to designate orientation of the halogen atom(s) relative to the phenoxy alcohol group.

After polymerization, appropriate work up of the polymers in accordance with preferred embodiments of the present invention may be achieved by any of a variety of known methods commonly employed in the field of synthetic polymers to produce a variety of useful articles with valuable physical and chemical properties, all derived from tissue compatible monomers. The useful articles can be shaped by conventional polymer-forming techniques such as extrusion, compression molding, injection molding, solvent casting, spin casting, wet spinning, combinations of two or more thereof, and the like. Shaped articles prepared from the polymers are useful, inter alia, as degradable biomaterials for medical implant applications. Such applications include the use of shaped articles as vascular grafts and stents.

Polymers according to the present invention also include polyethers, polyurethanes, poly(carbamates), poly(thiocarbonates), poly(carbonodithionates) and poly(thiocarbamates), which may be prepared from the diphenol compounds of the present invention in accordance with known methods.

Random or block copolymers of the polymers of the present invention with a poly(alkylene oxide) may be prepared according to the method disclosed in U.S. Pat. No. 5,658,995. The poly(alkylene oxide) is preferably a poly(ethylene glycol) block/unit typically having a molecular weight of less than 10,000 per unit. More typically, the poly(ethylene glycol) block/unit has a molecular weight less than 4000 per unit. The molecular weight is preferably between 1000 and 2000 per unit.

Alternatively, the block copolymers of the polymers of the present invention comprise a poly(alkylene oxide) macromer having the formula: 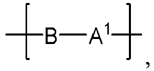
where B is -O-((CH₂)ₚ-O)_{q}- or -O-(CH₂CH(CH₃)-O)_{q}-;
p is an integer ranging from 1 to 6, preferably 1 to 3; more preferably 2;
q is 5 to 3000, preferably 0 to 300, more preferably 20 to 200, still more preferably 50 to 100;
and A¹ is selected from the group consisting of: 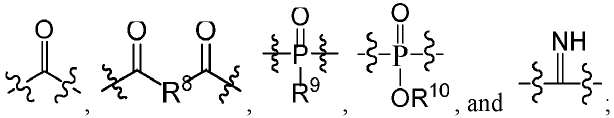
where R⁸ is selected from a bond, C₁-C₃₀ alkylene, C₂-C₃₀ alkenylene, C₂-C₃₀ alkynylene; C₁-C₃₀ heteroalkylene, C₂-C₃₀ heteroalkenylene, C₂-C₃₀ heteroalkynylene, C₇-C₃₀ heteroalkylarylene, C₈-C₃₀ heteroalkenylarylene, C₈-C₃₀ heteroalkynylarylene, C₇-C₃₀ alkylarylene, C₈-C₃₀ alkenylarylene, C₈-C₃₀ alkynylarylene, and C₂-C₃₀ heteroarylene; and R⁹ and R¹⁰ are each independently selected from H, C₁-C₃₀ alkyl, C₁-C₃₀ heteroalkyl, C₂-C₃₀ alkenyl, C₂-C₃₀ alkynyl, C₂-C₃₀ heteroalkenyl, and C₂-C₃₀ heteroalkynyl.

The molar fraction of poly(ethylene glycol) units in block copolymers may range from greater than zero to less than 1, and is typically greater than zero up to 0.5, inclusive. More preferably, the molar fraction is less than 0.25 and yet more preferably, less than 0.1. In a more preferred variations, the molar fraction may vary from greater than 0.001 to 0.08, and most preferably, between 0.025 and 0.035.

Unless otherwise indicated, the molar fractions reported herein are based on the total molar amount of poly(alkylene glycol) and non-glycol units in the polymers.

After polymerization, appropriate work up of the polymers in accordance with preferred embodiments of the present invention may be achieved by any of a variety of known methods commonly employed in the field of synthetic polymers to produce a variety of useful articles with valuable physical and chemical properties, all derived from tissue compatible monomers. The useful articles can be shaped by conventional polymer thermo-forming techniques such as extrusion and injection molding when the degradation temperature of the polymer is above the glass transition or crystalline melt temperature, or conventional non-thermal techniques can be used, such as compression molding, injection molding, solvent casting, spin casting, wet spinning. Combinations of two or more methods can be used. Shaped articles prepared from the polymers are useful, inter alia, as degradable biomaterials for medical implant applications.

Those skilled in the art will recognize that by appropriate selection of variable groups, embodiments of the compounds described above can be a hydroxyphenyl-alkanoic acid, such as desaminotyrosyl tyrosine (DAT), or a hydroxyphenylalkenoic acid. When the compound of the formula HX³-D¹-X⁴H is a diol, the two compounds may be reacted in an acid catalyzed Fischer esterification reaction, illustrated generally as follows:

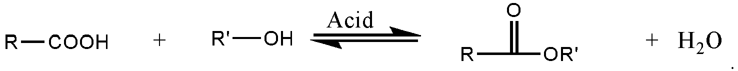

Because this reaction is reversible, removing water from the reaction mixture shifts the equilibrium to the right. Water removal is usually accomplished by way of azeotropic distillation; however other techniques known in the art may be employed as well. In instances where azeotropic distillation is desired, the solvent used for the reaction is preferably carefully chosen so that it forms an azeotropic mixture with water. Generally, solvents such as toluene, heptane, chloroform, tetrachloethylene are preferred.

The main advantage of this reaction is that primary and secondary alcohols form esters with carboxylic acids under acid catalysis, whereas the phenolic hydroxy groups are unreactive under these conditions. Thus the carboxylic acid groups of certain compounds, such as the 3-(4-hydroxyphenyl) propionic acid (DAT) and of 3-(3,5-diiodo-4-hydroxyphenyl) propionic acid (I₂DAT), can be reacted with primary or secondary alcohols while the phenolic groups remain intact.

### MEDICAL USES

Various embodiments of the polymer compositions described herein, preferably derived from tissue compatible monomers, may be used to produce a variety of useful articles with valuable physical and chemical properties. The useful articles can be shaped by conventional polymer thermo-forming techniques such as extrusion and injection molding when the degradation temperature of the polymer is above the glass transition or crystalline melt temperature(s), or conventional non-thermal techniques can be used, such as compression molding, injection molding, solvent casting, spin casting, wet spinning. Combinations of two or more methods can be used. Shaped articles prepared from the polymers are useful, inter alia, as biocompatible, biodegradable and/or bioresorbable biomaterials for medical implant applications.

In one embodiment, the medical device is a stent. It is contemplated that a stent may comprise many different types of forms. For instance, the stent may be an expandable stent. In another embodiment, the stent may be configured to have the form of a sheet stent, a braided stent, a self-expanding stent, a woven stent, a deformable stent, or a slide-and-lock stent. Stent fabrication processes may further include two-dimensional methods of fabrication such as cutting extruded sheets of polymer, via laser cutting, etch-ing, mechanical cutting, or other methods, and assembling the resulting cut portions into stents, or similar methods of three-dimensional fabrication of devices from solid forms.

In certain other embodiments, the polymers are formed into coatings on the surface of an implantable device, particularly a stent, made either of a polymer as described herein or another material, such as metal. Such coatings may be formed on stents via techniques such as dipping, spray coating, combinations thereof, and the like. Further, stents may be comprised of at least one fiber material, curable material, laminated material and/or woven material. The medical device may also be a stent graft or a device used in embolotherapy.

The highly beneficial combination of properties associated with preferred embodiments of the polymers described herein means these polymers are well-suited for use in producing a variety of resorbable medical devices besides stents, especially implantable medical devices that are preferably radiopaque, biocompatible, and have various times of bioresorption. For example the polymers are suitable for use in resorbable implantable devices with and without therapeutic agents, device components and/or coatings with and without therapeutic agents for use in other medical systems, for instance, the musculoskeletal or orthopedic system (e.g., tendons, ligaments, bone, cartilage skeletal, smooth muscles); the nervous system (e.g., spinal cord, brain, eyes, inner ear); the respiratory system (e.g., nasal cavity and sinuses, trachea, larynx, lungs); the reproductive system (e.g., male or female reproductive); the urinary system (e.g., kidneys, bladder, urethra, ureter); the digestive system (e.g., oral cavity, teeth, salivary glands, pharynx, esophagus, stomach, small intestine, colon), exocrine functions (biliary tract, gall bladder, liver, appendix, recto-anal canal); the endocrine system (e.g., pancreas/islets, pituitary, parathyroid, thyroid, adrenal and pineal body), the hematopoietic system (e.g., blood and bone marrow, lymph nodes, spleen, thymus, lymphatic vessels); and, the integumentary system (e.g., skin , hair, nails, sweat glands, sebaceous glands).

The polymers described herein can thus be used to fabricate wound closure devices, hernia repair meshes, gastric lap bands, drug delivery implants, envelopes for the implantation of cardiac devices, devices for other cardiovascular applications, noncardiovascular stents such as biliary stents, esophageal stents, vaginal stents, lungtrachea/bronchus stents, and the like.

In addition, the resorbable polymers are suitable for use in producing implantable, radiopaque discs, plugs, and other devices used to track regions of tissue removal, for example, in the removal of cancerous tissue and organ removal, as well as, staples and clips suitable for use in wound closure, attaching tissue to bone and/or cartilage, stopping bleeding (homeostasis), tubal ligation, surgical adhesion prevention, and the like. Applicants have also recognized that preferred embodiments of the polymers described herein are well-suited for use in producing a variety of coatings for medical devices, especially implantable medical devices.

Further, in some preferred embodiments, the present polymers may be advantageously used in making various resorbable orthopedic devices including, for example, radiopaque biodegradable screws (interference screws), radiopaque biodegradable suture anchors, and the like for use in applications including the correction, prevention, reconstruction, and repair of the anterior cruciate ligament (ACL), the rotator cuff/rotator cup, and other skeletal deformities.

Other devices that can be advantageously formed from preferred embodiments of the polymers described herein, include devices for use in tissue engineering. Examples of suitable resorbable devices include tissue engineering scaffolds and grafts (such as vascular grafts, grafts or implants used in nerve regeneration). The resorbable polymers may also be used to form a variety of devices effective for use in closing internal wounds. For example biodegradable resorbable sutures, clips, staples, barbed or mesh sutures, implantable organ supports, and the like, for use in various surgery, cosmetic applications, and cardiac wound closures can be formed.

Various devices useful in dental applications may advantageously be formed according to embodiments of the described herein. For example devices for guided tissue regeneration, alveolar ridge replacement for denture wearers, and devices for the regeneration of maxilla-facial bones may benefit from being radiopaque so that the surgeon or dentist can ascertain the placement and continuous function of such implants by simple X-ray imaging.

Preferred embodiments of the polymers described herein are also useful in the production of bioresorbable, inherently radiopaque polymeric embolotherapy products for the temporary and therapeutic restriction or blocking of blood supply to treat tumors and vascular malformations, e.g., uterine fibroids, tumors (i.e., chemoembolization), hemorrhage (e.g., during trauma with bleeding) and arteriovenous malformations, fistulas and aneurysms delivered by means of catheter or syringe. Details of embolotherapy products and methods of fabrication in which the polymers described herein may be employed are disclosed in U.S. Patent Publication No. 20050106119 A1. Embolotherapy treatment methods are by their very nature local rather than systemic and the products are preferably fabricated from the radio-opaque polymers described herein, to permit fluoroscopic monitoring of delivery and treatment.

The polymers described herein are further useful in the production of a wide variety of therapeutic agent delivery devices. Such devices may be adapted for use with a variety of therapeutics including, for example, pharmaceuticals (i.e., drugs) and/or biological agents as previously defined and including biomolecules, genetic material, and processed biologic materials, and the like. Any number of transport systems capable of delivering therapeutics to the body can be made, including devices for therapeutics delivery in the treatment of cancer, intravascular problems, dental problems, obesity, infection, and the like.

A medical device that comprises a polymeric material may include one or more additional components, e.g., a plasticizer, a filler, a crystallization nucleating agent, a preservative, a stabilizer, a photoactivation agent, etc., depending on the intended application. For example, in an embodiment, a medical device comprises an effective amount of at least one therapeutic agent and/or a magnetic resonance enhancing agent. Non-limiting examples of preferred therapeutic agents include a chemotherapeutic agent, a non-steroidal anti-inflammatory, a steroidal anti-inflammatory, and a wound healing agent. Therapeutic agents may be co-administered with the polymeric material. In a preferred embodiment, at least a portion of the therapeutic agent is contained within the polymeric material. In another embodiment, at least a portion of the therapeutic agent is contained within a coating on the surface of the medical device.

Non-limiting examples of preferred chemotherapeutic agents include taxanes, taxinines, taxols, paclitaxel, dioxorubicin, cis-platin, adriamycin and bleomycin. Non-limiting examples of preferred non-steroidal anti-inflammatory compounds include aspirin, dexamethasone, ibuprofen, naproxen, and Cox-2 inhibitors (e.g., Rofexcoxib, Celecoxib and Valdecoxib). Non-limiting examples of preferred steroidal anti-inflammatory compounds include dexamethasone, beclomethasone, hydrocortisone, and prednisone. Mixtures comprising one or more therapeutic agents may be used. Non-limiting examples of preferred magnetic resonance enhancing agents include gadolinium salts such as gadolinium carbonate, gadolinium oxide, gadolinium chloride, and mixtures thereof.

The amounts of additional components present in the medical device are preferably selected to be effective for the intended application. For example, a therapeutic agent is preferably present in the medical device in an amount that is effective to achieve the desired therapeutic effect in the patient to whom the medical device is administered or implanted. Such amounts may be determined by routine experimentation. In certain embodiments, the desired therapeutic effect is a biological response. In an embodiment, the therapeutic agent in the medical device is selected to promote at least one biological response, preferably a biological response selected from the group consisting of thrombosis, cell attachment, cell proliferation, attraction of inflammatory cells, deposition of matrix proteins, inhibition of thrombosis, inhibition of cell attachment, inhibition of cell proliferation, inhibition of inflammatory cells, and inhibition of deposition of matrix proteins. The amount of magnetic resonance enhancing agent in a medical devices is preferably an amount that is effective to facilitate radiologic imaging, and may be determined by routine experimentation.

The term "pharmaceutical agent", as used herein, encompasses a substance intended for mitigation, treatment, or prevention of disease that stimulates a specific physiologic (metabolic) response. The term "biological agent", as used herein, encompasses any substance that possesses structural and/or functional activity in a biological system, including without limitation, organ, tissue or cell based derivatives, cells, viruses, vectors, nucleic acids (animal, plant, microbial, and viral) that are natural and recombinant and synthetic in origin and of any sequence and size, antibodies, polynucleotides, oligonucleotides, cDNA's, oncogenes, proteins, peptides, amino acids, lipoproteins, glycoproteins, lipids, carbohydrates, polysaccharides, lipids, liposomes, or other cellular components or organelles for instance receptors and ligands. Further the term "biological agent", as used herein, includes virus, serum, toxin, antitoxin, vaccine, blood, blood component or derivative, allergenic product, or analogous product, or arsphenamine or its derivatives (or any trivalent organic arsenic compound) applicable to the prevention, treatment, or cure of diseases or injuries of man (per Section 351(a) of the Public Health Service Act (42 U.S.C. 262(a)). Further the term "biological agent" may include 1) "biomolecule", as used herein, encompassing a biologically active peptide, protein, carbohydrate, vitamin, lipid, or nucleic acid produced by and purified from naturally occurring or recombinant organisms, antibodies, tissues or cell lines or synthetic analogs of such molecules; 2) "genetic material" as used herein, encompassing nucleic acid (either deoxyribonucleic acid (DNA) or ribonucleic acid (RNA), genetic element, gene, factor, allele, operon, structural gene, regulator gene, operator gene, gene complement, genome, genetic code, codon, anticodon, messenger RNA (mRNA), transfer RNA (tRNA), ribosomal extrachromosomal genetic element, plasmagene, plasmid, transposon, gene mutation, gene sequence, exon, intron, and, 3) "processed biologics", as used herein, such as cells, tissues or organs that have undergone manipulation. The thera-peutic agent may also include vitamin or mineral substances or other natural elements.

For devices placed in the vascular system, e.g., a stent, the amount of the therapeutic agent is preferably sufficient to inhibit restenosis or thrombosis or to affect some other state of the stented tissue, for instance, heal a vulnerable plaque, and/or prevent rupture or stimulate endothelialization. The agent(s) may be selected from the group consisting of antiproliferative agents, anti-inflammatory, anti-matrix metalloproteinase, and lipid lowering, cholesterol modifying, anti-thrombotic and antiplatelet agents, in accordance with preferred embodiments of the present invention. In some preferred embodiments of the stent, the therapeutic agent is contained within the stent as the agent is blended with the polymer or admixed by other means known to those skilled in the art. In other preferred embodiments of the stent, the therapeutic agent is delivered from a polymer coating on the stent surface. In another preferred variation the therapeutic agent is delivered by means of no polymer coating. In other preferred embodiments of the stent, the therapeutic agent is delivered from at least one region or one surface of the stent. The therapeutic may be chemically bonded to the polymer or carrier used for delivery of the therapeutic of at least one portion of the stent and/or the therapeutic may be chemically bonded to the polymer that comprises at least one portion of the stent body. In one preferred embodiment, more than one therapeutic agent may be delivered.

In certain embodiments, any of the aforementioned devices described herein can be adapted for use as a therapeutic delivery device (in addition to any other functionality thereof). Controlled therapeutic delivery systems may be prepared, in which a therapeutic agent, such as a biologically or pharmaceutically active and/or passive agent, is physically embedded or dispersed within a polymeric matrix or physically admixed with a polymer described herein. Controlled therapeutic agent delivery systems may also be prepared by direct application of the therapeutic agent to the surface of an implantable medical device such as a bioresorbable stent device (comprised of at least one of the polymers described herein) without the use of these polymers as a coating, or by use of other polymers or substances for the coating.

Therapeutic agent delivery compounds may also be formed by physically blending the therapeutic agent to be delivered with the polymers described herein using conven-tional techniques well-known to those of ordinary skill in the art. For this therapeutic agent delivery embodiment, it is not essential that the polymer have pendent groups for covalent attachment of the therapeutic agent.

The polymer compositions described herein containing therapeutic agents, regardless of whether they are in the form of polymer conjugates or physical admixtures of polymer and therapeutic agent, are suitable for applications where localized delivery is desired, as well as in situations where a systemic delivery is desired. The polymer conjugates and physical admixtures may be implanted in the body of a patient in need thereof, by procedures that are essentially conventional and well-known to those of ordinary skill in the art.

The polyarylates can also be formed into drug delivery implants that degrade to release pharmacologically or biologically active agents within a predictable controlled release time. Such controlled drug delivery systems can be prepared by incorporating the active agents into the polymer chains as pendant side chains or by cross linking the pendant side chains to form a polymeric matrix into which the active agents are physically embedded or dispersed. Controlled drug delivery system implants can also be formed by physically admixing the polyarylates with a biologically or pharmacologically active agent. The foregoing procedures are essentially conventional and well-known to those of ordinary skill in the art.

For controlled drug delivery systems in which a biologically or pharmacologically active agent is physically embedded or dispersed into a polymeric matrix or physically admixed with a polyarylate, suitable biologically or pharmacologically active agents include in principle any active agent that has to be repeatedly administered over prolonged periods of time.

An advantage of using the radiopaque, bioresorbable polymers described herein in therapeutic agent delivery applications is the ease of monitoring release of a therapeutic agent and the presence of the implantable therapeutic delivery system. Because the radiopacity of the polymeric matrix is due to covalently attached halogen substituents, the level of radiopacity is directly related to the residual amount of the degrading therapeutic agent delivery matrix still present at the implant site at any given time after implantation. In preferred embodiments the rate of therapeutic release from the degrading therapeutic delivery system will be correlated with the rate of polymer resorption. In such preferred embodiments, the straight-forward, quantitative measurement of the residual degree of radio-opacity will provide the attending physician with a way to monitor the level of therapeutic release from the implanted therapeutic delivery system.

The following non-limiting examples set forth herein below illustrate certain aspects of the invention. All parts and percentages are by mole percent unless otherwise noted and all temperatures are in degrees Celsius unless otherwise indicated. All solvents were HPLC grade and all other reagents were of analytical grade and used as received, unless otherwise indicated.

### EXAMPLES

All reagents were purchased from commercial sources in pure form and were used as received. Solvents were of "HPLC" or "ACS reagent" grade.

Generally, the diphenolic monomers were prepared by Fisher-esterification of tyrosol with phenolic acids such as desaminotyrosine, 3,5-diiododesaminotyrosine or dicarboxylic acids (0.5 equivalents) by refluxing with catalytic amount of 4-toulenesulfonic acid in chloroform or 1,2-dichloroethane as the solvent. A modified Dean Stark trap was used to remove the water formed. The diphenolic monomers in the pure form or as appropriate mixtures were polymerized to the corresponding polycarbonates using triphosgene. The polymers were compression molded into films. The films were tested for mechanical properties and they generally showed high modulus, tensile strength, and elongation at break. Further details are provided below.

### Reference Example 1. Synthesis of 4-hydroxyphenethyl 3-(4-hydroxyphenyl)propanoate (DTy) and related diphenol monomers

Into a 500 mL round bottomed flask fitted with an overhead stirrer, and a modified Dean-stark trap for solvents heavier than water were added 10 g (72 mmol) of tyrosol, 13 g (78 mmol) of desaminotyrosine (DAT), 0.65 g (3.4 mmol) of 4-toluenesulfonic acid monohydrate, and 200 mL of 1,2-dichloroethane (DCE). A water-cooled reflux condenser was placed on top of the modified Dean-stark trap and the contents of the flask were heated to reflux while being stirred. The reaction was continued until approximately 1.4 mL of water collected in the modified Dean-stark trap above the DCE and the water collection essentially stopped (about 4 hours of reflux). The reaction mixture was cooled to room temperature when the crude product precipitated as off-white crystalline solid, which was dissolved in 100 mL of ethyl acetate and washed twice with 100 mL portions of 5% sodium bicarbonate solution. After drying over magnesium sulfate the organic layer was concentrated and precipitated with hexane. The resulting white crystalline solid was collected by filtration and dried in a vacuum oven at 25 °C. The product was characterized by elemental analysis, HPLC, and ¹H NMR.

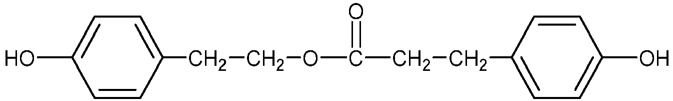

Using a similar procedure, 4'-hydroxyphenethyl-4-hydroxyphenyl acetate (HPTy, compound of Formula (V) where L¹ = L⁴ = bond, m = 2, n = 1, y1 = y2 = 0) was prepared by substituting 4-hydroxyphenyl acetic acid for desaminotyrosine. The product was characterized by hplc and ¹H NMR.

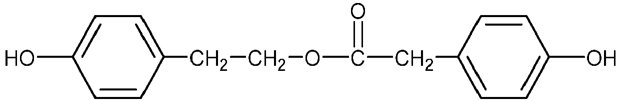

Using a similar procedure, 4-hydroxyphenethyl 2-(4-hydroxyphenoxy) acetate (compound of Formula (V) where L¹= bond, L⁴ = -O-, m = 2, n = 1, y1 = y2 = 0) is prepared by substituting 2-(4-hydroxyphenoxy)acetic acid for desaminotyrosine. Similar results are obtained.

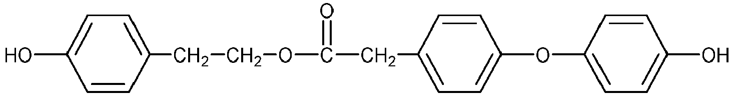

Using similar procedures, a monomer having the structure below (compound of Formula (VI) where Z = -NH-C(O)-CH₃, X¹ = I, y1 = 2, y2 = 0) is prepared by reacting N-acetyltyrosine with diiodotyrosol using a solvent or mixture of solvents in which the N-acetyl tyrosine is more soluble than in 1,2-dichloroethane. Similar results are obtained.

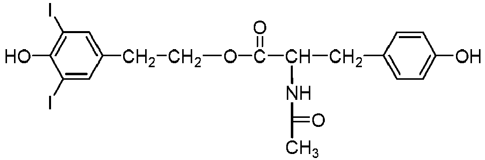

Using similar procedures, a monomer having the structure below (compound of Formula (VI) where Z = -NH-C(O)-CH₃, y1 = y2 = 0) is prepared by reacting N-acetyltyrosine with tyrosol using a solvent or mixture of solvents in which N-acetyl tyrosine is more soluble than in 1,2-dichloroethane. Similar results are obtained.

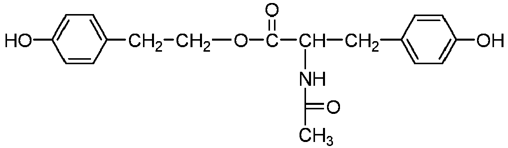

### Example 1. Synthesis of tyrosol ester of 4-hydroxybenzoic acid (tyrosyl(4-hydroxybenzoate), BTy)

Into a 250 mL round bottomed flask fitted with a Dean-Stark trap and an overhead stirrer is added 69.0 g ( 0.5 mol) of hydroxybenzoic acid, 66.3 g (0.49 mol) of tyrosol and 0.2 g of Sb₂O₃. The flask is heated in a n oil bath maintained at 210 °C while stirring with the overhead stirrer. The solid mixture melts into a clear liquid and water of reaction distilled over and collected in the Dean-Stark trap. When the water stops coming over the reaction was stopped. The reaction mixture was allowed to cool to room temperature. The ¹H NMR spectrum of the solid is in agreement with structure. The product is purified by recrystallization.

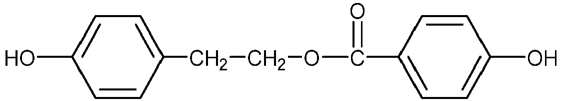

### Example 2A. Synthesis of 4-hydroxyphenethyl 3-(4-hydroxy-3,5-diiodophenyl)-propanoate (I2DTy) and related compounds

Into a 500 mL round bottomed flask fitted with an overhead stirrer, and a modified Dean-stark trap for solvents heavier than water were added 34.5 g (0.250 mol) **of tyrosol,** 102 g (0.244 mol) of 3-(4-hydroxy-3,5-diiodophenyl)propanoic acid (I₂DAT), 4.76 g (0.025 mol) of 4-toluenesulfonic acid monohydrate, and 500 mL of DCE. A water-cooled reflux condenser was placed on top of the modified Dean-stark trap and the contents of the flask were heated to reflux while being stirred. The reaction was continued until approximately 4.8 mL of water collected in the modified Dean-stark trap above the DCE and the water collection essentially stopped. The reaction mixture was allowed to cool to room temperature when the crude product precipitated as off-white crystals which was dried and then dissolved in 350 mL of tetrahydrofuran (thf). To this solution was added while stirring 1 L of 5% aqueous sodium bicarbonate solution stirred for 10 m and then allowed stand when the layers separated. The top layer was removed and discarded. The bottom layer was washed with two 500 mL portions of 5% aqueous sodium bicarbonate solution. I₂DTy precipitated as white crystalline solid. This was isolated by filtration and washed with 3 X 50 mL of deionized water. The product was dried under vacuum at 40 °C for 24 h and characterized by elemental analysis, HPLC, and ¹H NMR.

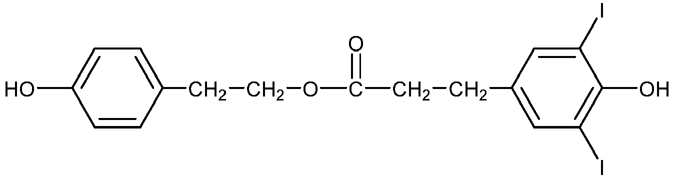

Using similar procedures, 4'-hydroxyphenethyl-2-(4-hydroxy-3,5-diiodophenyl)acetate (I₂HPTy) was prepared by substituting 2-(4-hydroxy-3-5-diiodophenyl)acetic acid for I₂DAT, and characterized by hplc and ¹H NMR.

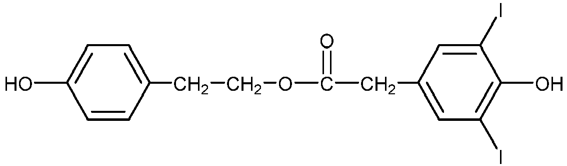

Using similar procedures, 4'-hydroxy-3',5'-diiodophenethyl-2-(4-hydroxy-3,5-diiodophenyl)propionate is prepared by substituting 4-(2-hydroxyethyl)-2,6-diiodophenol for tyrosol.

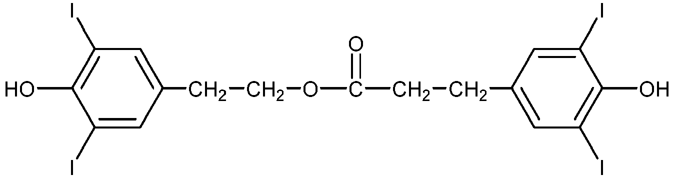

### Example 2B. Synthesis of 3',5'-diiodotyrosyl 3,5-diiodo(4-hydroxybenzoate), (I4BTy)

As described above, preparation of halogenated diphenolic monomers including hydroxybenzoic acid by reaction of halogenated precursors is problematic, due to halogen loss during reaction. Thus, by using a non-halogenated diphenolic HBA-tyrosol ester precursor in a halogenation reaction, a multiply-halogenated monomer is prepared. The tyrosyl(4-hydroxybenzoate), (BTy) prepared in Example 1 may be used.

Method 1: Tyrosyl(4-hydroxybenzoate) (64.6 g, 0.25 mol) was heated with 250 mL of 95% ethanol to reflux in 1 L round bottomed flask with an overhead stirrer under a N₂ atmosphere. The solid partially dissolved and to the suspension was added 139.7 g (0.55 mol) of iodine. Through an addition funnel 39 g (0.07 mol) of potassium iodate dissolved in 440 mL of deionized water was added to the reaction mixture over 15 min. The reaction mixture was heated to reflux for 2 h while stirring vigorously. The reaction mixture was then cooled to room temperature. The pink colored precipitate was isolated by filtration and washed with a 10% Na₂S₂O₃ solution followed by washing with water. The product was dried in a vacuum oven at 40 °C. The product was purified by recrystallization. Product was characterized by ¹H NMR, HPLC, and elemental analysis.

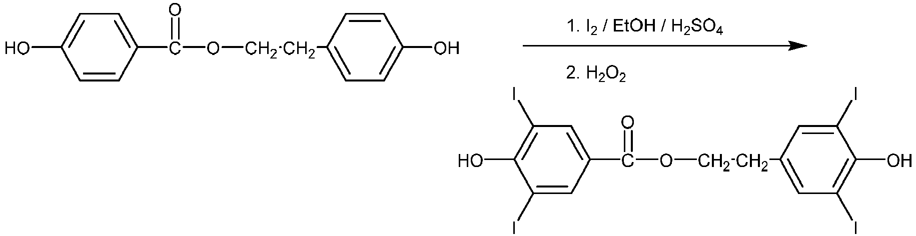

Method 2: In a 500 mL flask a mixture of 17.1 g of tyrosyl(4-hydroxybenzoate), 34 g of iodine, 100 mL of 95% ethanol, and 9 mL of sulfuric acid was stirred with a magnetic stirrer and heated to 60 °C using a water bath. To this was then added 21 mL of 30% aqueous H₂O₂ using an addition funnel over 30 min. After stirring for 30 min, the mixture was cooled and added to 400 mL of water with stirring. The precipitate was isolated by filtration and washed with 2 100 mL portions of acetic acid and then with 2 100 mL portions of acetone. The product was dried in a vacuum oven at 40 °C. It was characterized by ¹H NMR, HPLC, and elemental analysis.

### Reference Example 2. Synthesis of dityrosyl succinate

Into a 500 mL round bottomed flask fitted with an overhead stirrer, and a modified Dean-stark trap for solvents heavier than water were added 25.0 g (0.181 mol) of tyrosol, 9.56 g (0.088 mol) of succinic acid, 3.44 g (18.1 mmol) of 4-toluenesulfonic acid monohydrate, and 200 mL of DCE. A water-cooled reflux condenser was attached to the top of the modified Dean-stark trap and the contents of the flask were heated to reflux while being stirred. The reaction was continued until approximately 3.2 mL of water collected in the modified Dean-stark trap above the DCE and the water collection essentially stopped. The reaction mixture was allowed to cool to room temperature while stirring was continued. The product that precipitated was isolated by filtration and washed with 2X50 mL of DCE. ¹H NMR showed residual PTSA and tyrosol. For purification the solid was stirred with 150 mL of aqueous 5% NaHCO₃ for 3 h using overhead stirrer. The product was isolated by filtration and washed with 3 X 50 mL of DIwater and then dried in the vacuum oven for 24 h at 50 °C. The product was dried under vacuum at 40 °C for 24 h and characterized by elemental analysis, HPLC, and ¹H NMR spectroscopy.

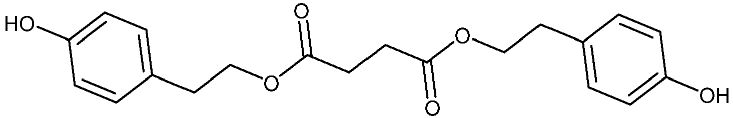

### Reference Example 3. Synthesis of dityrosyl Oxalate

Into a 500 mL round bottomed flask fitted with an overhead stirrer, and a modified Dean-stark trap for solvents heavier than water were added 25.0 g (0.181 mol) of tyrosol, 8.00 g (0.088 mol) of Oxalic acid, 3.44 g (18.1 mmol) of 4-toluenesulfonic acid monohydrate, and 200 mL of 1,2-DCE (dichloroethane). A water-cooled reflux condenser was attached to the top of the modified Dean-stark trap and the contents of the flask were heated to reflux while being stirred. The reaction was continued until approximately 3.2 mL of water collected in the modified Dean-stark trap above the DCE and the water collection essentially stopped. The reaction mixture was allowed to cool to room temperature while stirring was continued. The product that precipitated was isolated by filtration and washed with 2 X 50 mL of DCE. For purification the solid was stirred with 150 mL of aqueous 5% NaHCO3 for 3 h using overhead stirrer. The product was isolated by filtration and washed with 3 X 50 mL of DI (deionized) water and then dried in the vacuum oven for 24 h at 50 °C. The product was dried under vacuum at 40 °C for 24 h and characterized by elemental analysis, HPLC, and ¹H NMR spectroscopy.

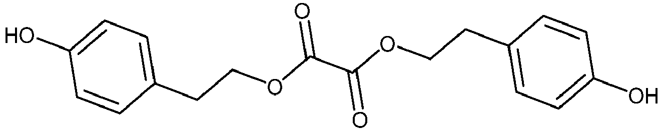

### Reference Example 4. Polymerization of DTy and HPTy using triphosgene

In a 500 mL 3-necked round-bottomed flask equipped with a mechanical stirrer, and a liquid addition device were placed 8.0 g (0.035 mol) of DTy, 9.5 g (0.12 mol) of pyridine, 70 mL of dichloromethane (DCM) and stirred for 15 min to get a clear solution. Triphosgene (3.6 g, 0.036 mol) was dissolved in 15 mL of DCM and the solution was introduced into the reaction flask over 2-3 hours. After the addition was complete, 100 mL of water was added to the reaction mixture and stirred for 5 min. After allowing the layers to separate, the top aqueous layer was removed and discarded. The washing as above was repeated with two additional 100 mL portions of DI water. The reaction mixture was then precipitated with 120 mL of IPA. The resulting gel was ground twice with 150 mL portions of IPA in 1 L laboratory blender. The product was isolated by vacuum filtration and dried in a vacuum oven at 80 °C for 24 h. The polymer had a HPSEC polystyrene equivalent molecular weight of 160 Kda (THF as mobile phase). The polymer was semi-crystalline with a Tg of 51 °C and a Tm of 181 °C. On compression molding at 220 °C, it gave films which were transparent on rapid cooling and translucent when cooled slowly. The tensile modulus, tensile stress at yield, and elongation at break were respectively 210 ksi, 5 ksi and 500%.

Using similar procedures, HPTy (4'-hydroxyphenethyl-4-hydroxyphenyl acetate, such as obtained in accordance with Reference Example 1) was polymerized to obtain poly(HPTy carbonate) with an HPSEC polystyrene equivalent Mw of 140 Kda and a Tg of 55 °C.

### Example 3. Polymerization of I₂DTy and I₂HPTy using triphosgene

In a 500 mL 3-necked round-bottomed flask equipped with a mechanical stirrer, and a controlled liquid addition device were placed 25 g (0.046 mol) of I₂DTy, 14.3 g (0.181 mol) of pyridine, 200 mL of DCM and stirred for 15 min to get a clear solution. Triphosgene (5.1 g, 0.052 mol of phosgene) was dissolved in 20 mL of DCM and the solution was to the reaction flask over 2-3 hours. After the addition was complete, 250 mL of water was added to the reaction mixture and stirred for 5 min. After allowing the layers to separate, the top aqueous layer was removed and discarded. The washing was repeated with two additional 250 mL portions of DI water. The reaction mixture was then precipitated with 350 mL of IPA. The resulting gel was ground twice with 200 mL portions of IPA in a 1 L laboratory blender. The product was isolated by vacuum filtration and dried in a vacuum oven at 80 °C for 24 h. The polymer had a HPSEC polystyrene equivalent molecular weight of 176 Kda (THF as mobile phase) and glass transition temperature (Tg) of 112 °C. Compression molding at 205 °C gave a uniform transparent film which gave tensile modulus, tensile stress at yield, and elongation at break respectively of 230 ksi, 9.2 ksi and 220%. Using similar procedures, I₂HPTy (obtained in accordance with Example 2) was polymerized to obtain poly(I₂HPTy carbonate).

### Refernce Example 5. Preparation of Poly(I₂DTy-co-10weight% PEG2K carbonate)

In a 250 mL 3-necked round-bottomed flask equipped with a mechanical stirrer, and a liquid addition device were placed 9.0 g (0.017 mol) of I₂DTy, 1.01 g of PEG2000, 5.4 g (0.068 mol) of pyridine, and 65 mL of DCM and stirred for 15 min to get a clear solution. Triphosgene (2.0 g, 0.020 mol of phosgene) was dissolved in 10 mL of DCM and the solution was introduced into the reaction flask over 2-3 hours. After the addition was complete, 100 mL of water was added to the reaction mixture and stirred for 5 min. After allowing the layers to separate, the top aqueous layer was removed and discarded. The washing was repeated with two additional 100 mL portions of DI water. The reaction mixture was then precipitated with 100 mL of IPA. The resulting gel was ground twice with 150 mL portions of IPA in 1 L laboratory blender. The product was isolated by vacuum filtration and dried in a vacuum oven at 50 °C. The polymer had a HPSEC polystyrene equivalent molecular weight of 250 Kda (THF as mobile phase) and glass transition temperature (Tg) of 64 °C and gave a clear film on compression molding at 205 °C. The tensile stress at yield, the tensile modulus and elongation at break respectively were 7.1 ksi, 235 ksi and 350%.

### Reference Example 6. Preparation of Poly(I₂DTy-co-5 weight% PEG2K carbonate)

In a 250 mL 3-necked round-bottomed flask equipped with a mechanical stirrer, and a liquid addition device were placed 10 g (0.019 mol) of I₂DTy, 0.535 g of PEG2000, 5.9 ml (0.073 mol) of pyridine, and 62 mL of DCM and stirred for 15 min to get a clear solution. Triphosgene (2.1 g, 0.021 mol of phosgene) was dissolved in 10 mL of DCM and the solution was introduced into the reaction flask over 2-3 hours. After the addition was complete, 100 mL of water was added to the reaction mixture and stirred for 5 min. After allowing the layers to separate, the top aqueous layer was removed and discarded. The washing was repeated with two additional 100 mL portions of DI water. The reaction mixture was then precipitated with 100 mL of IPA. The resulting gel was ground twice with 150 mL portions of IPA in 1 L laboratory blender. The product was isolated by vacuum filtration and dried in a vacuum oven at 50 °C. The polymer had a HPSEC polystyrene equivalent molecular weight of 200 Kda (THF as mobile phase) and glass transition temperature (Tg) of 84 °C. Compression molding at 205 °C gave a uniform transparent film which gave tensile modulus, tensile stress at yield , and elongation at break respectively of 232 ksi, 8.2 ksi and 70%.

### Reference Example 7. Preparation of Poly(I₂DTy-co-10weight% PTMC5K carbonate)

In a 250 mL 3-necked round-bottomed flask equipped with a mechanical stirrer, and a liquid addition device were placed 9.0 g (0.017 mol) of I₂DTy, 1.00 g of poly(trimethylene carbonate) of Mn 5000 (PTMC5K), 5.5 ml (0.068 mol) of pyridine, and 65 mL of DCM and stirred for 15 min to get a clear solution. Triphosgene (1.9 g, 0.019 mol of phosgene) was dissolved in 10 mL of DCM and the solution was introduced into the reaction flask over 2-3 hours. After the addition was complete, 100 mL of water was added to the reaction mixture and stirred for 5 min. After allowing the layers to separate, the top aqueous layer was removed and discarded. The washing was repeated with two additional 100 mL portions of DI water. The reaction mixture was then precipitated with 100 mL of IPA. The resulting gel was ground twice with 150 mL portions of IPA in 1 L laboratory blender. The product was isolated by vacuum filtration and dried in a vacuum oven at 50 °C. The polymer had a HPSEC polystyrene equivalent molecular weight of 250 Kda (THF as mobile phase) and glass transition temperature (Tg) of 101 °C. Compression molding at 205 °C gave a uniform transparent film which gave tensile modulus, tensile stress at yield, and elongation at break respectively of 201 ksi, 7.4 ksi and 120%.

### Reference Example 8. Preparation of Poly(I₂DTy-co-5 weight% PTMC5K carbonate)

In a 250 mL 3-necked round-bottomed flask equipped with a mechanical stirrer, and a liquid addition device were placed 10 g (0.019 mol) of I₂DTy, 0.53 g of PTMC5K, 5.9 ml (0.073 mol) of pyridine, and 65 mL of DCM and stirred for 15 min to get a clear solution. Triphosgene (2.1 g, 0.021 mol of phosgene) was dissolved in 10 mL of DCM and the solution was introduced into the reaction flask over 2-3 hours. After the addition was complete, 100 mL of water was added to the reaction mixture and stirred for 5 min. After allowing the layers to separate, the top aqueous layer was removed and discarded. The washing was repeated with two additional 100 mL portions of DI water. The reaction mixture was then precipitated with 100 mL of IPA. The resulting gel was ground twice with 150 mL portions of IPA in 1 L laboratory blender. The product was isolated by vacuum filtration and dried in a vacuum oven at 50 °C. The polymer had a HPSEC polystyrene equivalent molecular weight of 225 Kda (THF as mobile phase) and glass transition temperature (Tg) of 106 °C. Compression molding at 205 °C gave a uniform transparent film which gave tensile modulus, tensile stress at yield, and elongation at break respectively of 266 ksi, 8.4 ksi and 185%.

### Reference Example 9. Synthesis of di-ester of 1,3-propanediol with I₂DAT (PrD-di I₂DAT)

Into a 500 mL round-bottomed flask equipped with an overhead stirrer, a Dean-Stark trap and a thermometer were added 3.04 g (0.040 mol) of 1,3-propanediol, 33.8 g (0.081 mol) of 3,5-diiododesaminotyrosyl tyrosine ethyl ester (I₂DAT), 0.76 g (4.0 mmol) of p-toluenesulfonic acid, and 200 mL of 1,2-dichloroethane. The flask was heated using a heating mantle, while stirring with the overhead stirrer so that 1,2-dichloroethane and water distilled over into the Dean-Stark trap. The heating continued until the water collection stopped (about 1.45 mL of water was collected). The reaction mixture was allowed to cool to 50 °C and then evaporated to dryness. To the residue 175 mL of acetonitrile was added and stirred at room temperature for 4 h. The crystalline solid that separated was isolated by filtration and washed with acetonitrile. The Off-white crude product was collected and dried.

The crude PrD-di **I₂DAT** obtained above (98% pure by HPLC) was stirred with 175 mL of acetonitrile for 4 h using a overhead stirrer at 200 rpm. The product precipitated as almost colorless powder, which showed a purity of ca 98-99% by HPLC. For further purification the product was dissolved in acetonitrile (10 mL/g) and stirred with Norit (10 mg of Norit /g of product). The hot solution was filtered to remove Norit and then cooled in ice-water bath for recrystallization when colorless powder was obtained (purity >99.5% by HPLC). The product was dried in vacuum oven at 40 °C. The product had a melting point of 88 °C (by DSC) and the elemental analysis and ¹H NMR spectrum were in agreement with the structure. Further purification can be achieved by column chromatography on silica gel.

### Reference Example 10. Preparation of Poly(PrD-di I₂DAT-co-10 weight% tyrosol carbonate)

In a 1 L 3-necked round-bottomed flask equipped with a mechanical stirrer, and a liquid addition device were placed 25 g (0.029 mol) of PrD-di I₂DAT, 2.78 g (0.020 mol) of tyrosol, 15.4 ml (0.19 mol) of pyridine, and 170 mL of DCM and stirred for 15 min to get a clear solution. Triphosgene (5.4 g, 0.055 mol of phosgene) was dissolved in 20 mL of DCM and the solution was introduced into the reaction flask over 2-3 hours. After the addition was complete, the 200 mL of water was added to the reaction mixture and stirred for 5 min. After allowing the layers to separate, the top aqueous layer was removed and discarded. The washing was repeated with two additional 200 mL portions of DI water. The reaction mixture was then precipitated with 300 mL of IPA. The resulting gel was ground twice with 200 mL portions of IPA in 1 L laboratory blender. The product was isolated by vacuum filtration and dried in a vacuum oven at 80 °C. The polymer had a HPSEC polystyrene equivalent molecular weight of 200 Kda (THF as mobile phase) and glass transition temperature (Tg) of 90 °C. ¹H NMR spectrum of the polymer was in agreement with the structure. Compression molding at 205 °C gave a uniform transparent film which gave tensile modulus, tensile stress at yield (σ), and elongation at break respectively of 260 ksi, 9.7 ksi and 220%. Using similar procedures copolymers with 5%, and 15% tyrosol were prepared as follows:

| % tyrosol | Tg, °C | σ, ksi | Modulus, ksi | Elongation, % |
|---|---|---|---|---|
| 5 | 104 | 9.8 | 254 | 41 |
| 15 | 90 | 9.5 | 244 | 164 |

As will be understood by a person of ordinary skill in the art, since triphogene is added slowly into the mixture of the reactants PrD-di I₂DAT and tyrosol, the poly(PrD-di I₂DAT-co-tyrosol carbonate) product is composed of mainly polymer molecules having randomly-ordered PrD-di **I₂DAT** and tyrosol units connected through carbonate (-OC(O)O-) linkers. That is, two adjacent units could include PrD-di **I₂DAT** and PrD-di I₂DAT, PrD-di I₂DAT and tyrosol, or tyrosol and tyrosol. Given the unsymmetrical structure of tyrosol, it can be connected with a PrD-di I₂DAT unit using either "head" (i.e., "phenoxy" moiety) or "tail" (i.e., the "ethylenoxy" moiety). Any two adjacent units formed from tyrosol itself can be in any of the "head-head", "head-tail" or "tail-tail" arrangements. In this Example, without intending to be bound by theory, since the PrD-di I₂DAT was used in molar excess, the polymer molecules likely do not contain a large amount of long strings of "tyrosolcarbonate-tyrsol" units linked to each other. On the other hand, if there is a large excess of tyrosol relative to the PrD-di I₂DAT in the reaction mixture, tyrosol may have more opportunity to link with each other to give relatively long strings of such linkages.

### Reference Example 11. Poly(tyrosol carbonate)

In a 500 mL 3-necked round-bottomed flask equipped with a mechanical stirrer, and a liquid addition device were placed 10 g (0.073 mol) of tyrosol, 24 ml (0.298 mol) of pyridine, 200 mL of DCM and stirred for 15 min to get a clear solution. Triphosgene (7.7 g, 0.078 mol of phosgene) was dissolved in 25 mL of DCM and the solution was introduced into the reaction flask over 2-3 hours. After the addition was complete, 250 mL of water was added to the reaction mixture and stirred for 5 min. After allowing the layers to separate, the top aqueous layer was removed and discarded. The washing was repeated with two additional 250 mL portions of DI water. The reaction mixture was then precipita-ted with 300 mL of IPA. The resulting gel was ground twice with 200 mL portions of IPA in 1 L laboratory blender. The product was isolated by vacuum filtration and dried in a vacuum oven at 60 °C. The polymer had a HPSEC polystyrene equivalent molecular weight of 126 Kda (THF as mobile phase) and glass transition temperature (Tg) of 58 °C. Compression molding at 195 °C gave a uniform transparent film which gave tensile modu-lus, tensile stress at yield, and elongation at break respectively of 191 ksi, 5 ksi and 450%.

### Reference Example 12. Low molecular weight Poly(tyrosol carbonate)

In a 250 mL 3-necked round-bottomed flask equipped with a mechanical stirrer, and a liquid addition device were placed 10 g (0.073 mol) of tyrosol, 22 ml (0.277 mol) of pyridine, 60 mL of DCM and stirred for 15 min to get a clear solution. Triphosgene (7.0 g, 0.071 mol of phosgene) was dissolved in 25 mL of DCM and the solution was introduced into the reaction flask over 2-3 hours. After the addition was complete, the 100 mL of 0.2 M aqueous HCl was added to the reaction mixture and stirred for 5 min. After allowing the layers to separate, the top aqueous layer was removed and discarded. The washing was repeated with three additional 100 mL portions of 0.2 M aqueous HCl. The reaction mixture was then dried over anhydrous magnesium sulfate and then precipitated with 100 mL of hexane. The resulting viscous oil was stirred with 200 mL of fresh hexane until the product solidified into a white solid. The product was transferred to a glass dish dried in a vacuum oven at 60 °C. The polymer had a HPSEC polystyrene equivalent Mw of 7500 da and Mn of 5700 da (THF as mobile phase) and glass transition temperature (Tg) of 48 °C. A number of oligomers and polymers ranging in Mw from 750 da to 40,000 da were prepared using this method.

### Reference Example 13. Preparation of multi-block Poly(PrD-di I₂DAT -co-10 weight% tyrosol carbonate)

In a 1 L 3-necked round-bottomed flask equipped with a mechanical stirrer, and a liquid addition device were placed 25 g (0.029 mol) of PrD-di I₂DAT, 2.78 g (0.49 mmol) of oligo(tyrosol carbonate) with Mn of 5700 da, 15.4 ml (0.19 mol) of pyridine, and 170 mL of DCM and stirred for 15 min to get a clear solution. Triphosgene (3.3 g, 0.055 0.034 mol of phosgene) was dissolved in 20 mL of DCM and the solution was introduced into the reaction flask over 2-3 hours. After the addition was complete, the reaction mixture was stirred for 15 min. To the viscous reaction mixture 200 mL of water was added and stirred for 5 min. After allowing the layers to separate, the top aqueous layer was removed and discarded. The washing was repeated with two additional 200 mL portions of DI water. The reaction mixture was then precipitated with 300 mL of IPA. The resulting gel was ground twice with 200 mL portions of IPA in 1 L laboratory blender. The product was isolated by vacuum filtration and dried in a vacuum oven at 80 °C. The polymer had a HPSEC polystyrene equivalent molecular weight of 200 Kda (THF as mobile phase) and glass transition temperature (Tg) of 90 °C. ¹H NMR spectrum of the polymer was in agreement with the structure. The ¹H NMR spectrum of this polymer was significantly different from the random copolymer obtained as in reference example 11, indicative of the blockiness of the tyrosol recurring units.

### Reference Example 14. Preparation of Poly(PrD-di I₂DAT -co-10 weight% DTy carbonate)

In a 1 L 3-necked round-bottomed flask equipped with a mechanical stirrer, and a liquid addition device were placed 25 g (0.029 mol) of PrD-di I₂DAT, 2.78 g (0.010 mol) of DTy, 15.4 ml (0.19 mol) of pyridine, and 170 mL of DCM and stirred for 15 min to get a clear solution. Triphosgene (4.3 g, 0.044 mol of phosgene) was dissolved in 20mL of DCM and the solution was introduced into the reaction flask over 2-3 hours. After the addition was complete, the 200 mL of water was added to the reaction mixture and stirred for 5 min. After allowing the layers to separate, the top aqueous layer was removed and discarded. The washing was repeated with two additional 200 mL portions of DI water. The reaction mixture was then precipitated with 300 mL of IPA. The resulting gel was ground twice with 200 mL portions of IPA in 1 L laboratory blender. The product was isolated by vacuum filtration and dried in a vacuum oven at 80 °C. The polymer had a HPSEC polystyrene equivalent molecular weight of 200 Kda (THF as mobile phase) and glass transition temperature (Tg) of 95 °C. ¹H NMR spectrum of the polymer was in agreement with the structure. Compression molding at 205 °C gave a uniform transparent film which gave tensile modulus, ultimate tensile stress, and elongation at break respectively of 280 ksi, 10 ksi and 200%.

### Reference Example 15. Preparation of Tyrosol or Analog-based Alternating Polycarbonates

Alternating polymers having regular sequences of tail-tail, head-head, and/or head-tail configurations are disclosed. These polymers are distinctly different from random polymers having no specific order of tail-tail, head-head, and/or head-tail configurations. Specifically, polycarbonates derived from tyrosol, have three types of carbonate bonds: aromatic-aromatic (also referred to as head-head), mixed aromatic-aliphatic (also referred to as head-tail), and aliphatic-aliphatic (also referred to as tail-tail) as shown below: 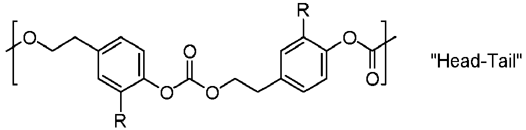 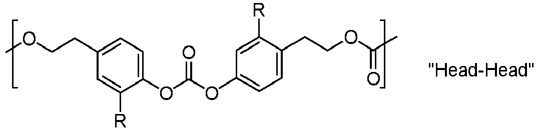 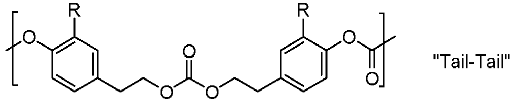 R = H (tyrosol) or OMe (homovanillyl alcohol)

Polymers with random sequences of H-H, H-T, or T-T backbone linkages can have distinctly different properties from those having a regular sequence of backbone linkages.

To create alternating polymers with a regular, alternating sequence of H-H and T-T bonds, the monomer was reacted with itself to form a dimer. Then, the dimer was subjected to a polymerization reaction. In this example, aliphatic dityrosol carbonate and aliphatic tyrosol chloroformate were used as monomers for polycarbonate synthesis. Aliphatic dityrosol carbonate introduces an enzymatic cleavage site due to the flexibility and steric accessibility of the aliphatic carbonate bond. Reaction steps are outlined below.

### (A) Synthesis of tyrosol chloroformate

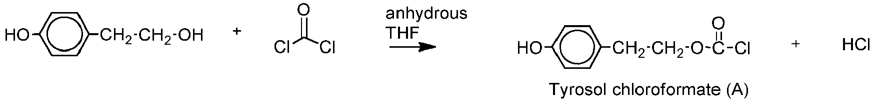

Tyrosol was placed in a three-necked flask equipped with an overhead stirrer under inert atmosphere. Anhydrous tetrahydrofuran was added from a syringe and a solution was obtained while the mixture was stirred. The solution was constantly cooled with an ice/water bath. Triphosgene was dissolved in anhydrous tetrahydrofuran and added drop-wise to the reaction vessel. Aliphatic tyrosol chloroformate was obtained over the course of one hour. Most of the solvent was evaporated to prepare for the work-up. Methylene chloride was added to dissolve the residue and excess tyrosol was filtered off. The solution was cooled in an ice/water bath. Cooled deionized water was added to remove most of the HCl built up during the reaction. The two layers were separated and the organic phase was dried over magnesium sulfate. The solvent was evaporated, and after drying under vacuum aliphatic tyrosol chloroformate was obtained as an oil.

### (B) Synthesis of aliphatic dityrosol carbonate

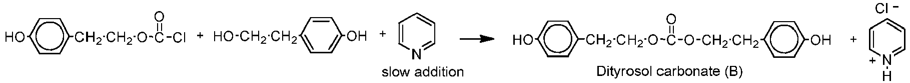

Aliphatic tyrosol chloroformate (A) and tyrosol were dissolved in anhydrous tetrahydrofuran under nitrogen atmosphere and cooled with an ice/water bath. One equivalent of pyridine was added drop-wise using a syringe pump over the course of twelve hours. Then the solvent was evaporated, and the residue dissolved in methylene chloride. The organic phase was washed 4 times with dilute HCl, 4 times with 5% (w/v) aqueous bicarbonate and twice with brine. The organic layer was dried over magnesium sulfate. After drying dityrosol carbonate was obtained as a white solid.

### (C) Synthesis of poly(tyrosol carbonate) with alternating carbonate bond sequence

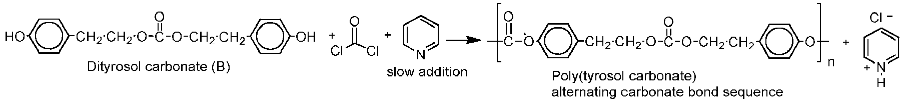

Aliphatic dityrosol carbonate is dissolved in methylene chloride under nitrogen atmosphere. Triphosgene is dissolved in methylene chloride and added drop-wise to the reaction mixture. After the triphosgene addition, pyridine is added drop-wise to the reaction mixture over the course of several hours. Poly(tyrosol carbonate) with alternating carbonate bond sequence is obtained by standard a workup procedure.

### (D) Synthesis of polytyrosol with controlled carbonate bond sequence

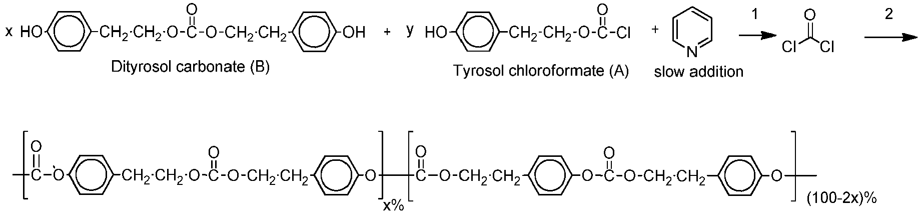

Dityrosol carbonate (x equivalents) and tyrosol carbonate (y equivalents) are dissolved in anhydrous tetrahydrofurane and cooled in dry ice/isopropanol bath. Pyridine is added drop-wise over the course of several hours in step 1. Then triphosgene dissolved in anhydrous tetrahydrofuran is added drop-wise into the reaction mixture. The poly(tyrosol carbonate) with controlled composition of carbonate bonds is obtained through a standard work-up procedure.

### Reference Example 16. Preparation of poly(PrDI₂DAT-co-9%tyrosol-co-1%PEG1K carbonate)

In a 1 L 3-necked round-bottomed flask equipped with a mechanical stirrer, and a liquid addition device were placed 45 g (51 mmol) of PrD-di I₂DAT, 4.5 g (33 mol) of tyrosol, 0.5 g (0.50 mmol) of PEG1000, 25 g (320 mmol) of pyridine, and 305 mL of DCM and stirred for 15 min to get a clear solution. Triphosgene (8.6 g, 87 mmol of phosgene) was dissolved in 32 mL of DCM and the solution was introduced into the reaction flask over 2-3 hours. After the addition was complete, the reaction mixture was quenched with a mixture of 135 mL of THF and 15 mL of water. 350 mL of water was added to the reaction mixture and stirred for 5 min. After allowing the layers to separate, the top aqueous layer was removed and discarded. The washing was repeated with two additional 350 mL portions of DI water. The reaction mixture was then precipitated with 500 mL of acetone. The resulting gel was stirred with 500 mL of IPA when the gel broke up into fine particles. The particles were ground twice, isolated by filtration and dried in a vacuum oven at 80 °C. The polymer had a Mw of 400 Kda and glass transition temperature (Tg) of 92 °C. ¹H NMR spectrum of the polymer was in agreement with the structure. Compression molding at 190 °C gave a uniform transparent film which gave tensile modulus, tensile stress at yield, and elongation at break of 240 ksi, 9.1 ksi, and 106% respectively.

### Reference Example 17. Preparation of poly(I₂DTy-co-10%tyrosol carbonate)

In a 1 L 3-necked round-bottomed flask equipped with a mechanical stirrer, and a liquid addition device were placed 45 g (0.084 mol) of I₂DTy, 5 g (0.036 mol) of tyrosol, 35.5 g (0.45 mol) of pyridine, and 300 mL of DCM and stirred for 15 min to get a clear solution. Triphosgene (12.3 g, 0.125 mol of phosgene) was dissolved in 32 mL of DCM and the solution was introduced into the reaction flask over 2-3 hours. After the addition was complete, the reaction mixture was quenched with a mixture of 135 mL of THF and 15 mL of water. 350 mL of water was added to the reaction mixture and stirred for 5 min. After allowing the layers to separate, the top aqueous layer was removed and discarded. The washing was repeated with two additional 350 mL portions of DI water. The reaction mixture was then precipitated with 600 mL of IPA. The resulting gel was ground twice in a 4 L high speed blender. The precipitate obtained was isolated by filtration and dried in a vacuum oven at 80 °C. The polymer had a Mw of 318 Kda and a glass transition temperature (Tg) of 100 °C. ¹H NMR spectrum of the polymer was in agreement with the structure. Compression molding at 190 °C gave a uniform transparent film. Using similar procedures a copolymer with 15% tyrosol was prepared. The properties of the polymers are set forth below:

| % tyrosol | Tg, °C | σ, ksi | Modulus, ksi | Elongation, % |
|---|---|---|---|---|
| 10 | 100 | 8.7 | 234 | 239 |
| 15 | 82 | 9.0 | 240 | 217 |

### Reference Example 18. Preparation of PLLAdiol using ethylene glycol as initiator (EGPLLAD7K).

Into a 250 mL round bottomed flask were transferred 1.29 g (0.02 mol) of ethylene glycol, 1.44 tg (3.6 mmol) of Sn(II)octoate and 144.1 g (1.0 mol) of L-lactide. A large egg-shaped stir bar was introduced into the flask. The flask was maintained under a positive pressure of nitrogen and then immersed into an oil bath maintained at 110 °C and after heating for 1 h the lactide melted. The temperature was raised to 140 °C and heated with stirring for 4 h. The flask was then removed from the oil bath and allowed to cool to room temperature. To the flask 350 mL of DCM was added and stirred overnight to dissolve the polymer. The polymer solution was slowly added to 1 L of heptane with stirring. The polymer precipitated as white crystalline powder which was isolated by filtration. The precipitate was washed with 250 mL of acetonitrile to remove any unreacted lactide. The product was dried in a vacuum oven at 40 °C for 24 h. DSC showed a Tg of 47 °C and melting points at 134 °C (5 J/g) and 148 °C (15.5 J/g). PrDPLLAD7K was similarly prepared using 1,3-propanediol as the initiator instead of ethylene glycol.

### Reference Example 19. Preparation of poly(PrD-di I₂DAT-co-50%EGPLLAD7K carbonate).

In a 1 L 3-necked round-bottomed flask equipped with a mechanical stirrer, and a liquid addition device were placed 30 g (0.034 mol) of PrD-di I₂DAT, 30 g (0.004 mol) of EGPLLAD7K, 11.4 g (0.145 mol) of pyridine, and 360 mL of chloroform and stirred for 15 min to get a clear solution (the solution was slightly cloudy). Triphosgene (3.96 g, 0.04 mol of phosgene) was dissolved in 12 mL of chloroform and the solution was introduced into the reaction flask over 2-3 hours. After the addition was complete, the reaction mixture was quenched with a mixture of 135 mL of THF and 15 mL of water. 350 mL of water was added to the reaction mixture and stirred for 5 min. After allowing the layers to separate, the top aqueous layer was removed and discarded. The washing was repeated with two additional 350 mL portions of DI water. The reaction mixture was then precipitated with 700 mL of IPA. The resulting gel was ground with 550 mL twice in a 4 L blender. The product was isolated by filtration and dried in a vacuum oven at 80 °C. ¹H NMR spectrum of the polymer was in agreement with the structure. Compression molding at 190 °C of the obtained 50% EGPLLAD polymer gave a uniform transparent film.

Using similar procedures, copolymers containing 20% and 65% EGPLLAD were also prepared. The physical properties of the three polymer samples are set forth below. Other polymers having different physical properties can be prepared by routine experimentation informed by the guidance provided herein, e.g., by appropriate selection of comonomer content, polymer molecular weight and film preparation procedures.

| % EGPLLAD | Tg, °C | σ, ksi | Modulus, ksi | Elongation, % |
|---|---|---|---|---|
| 20 | 60 and 110 | 9.4 | 262 | 6 |
| 50 | Tg = 61 | 8.0 | 274 | 162 |
| | Tm =150 | | | |
| 65 | Tg = 62 | 7.0 | 295 | 5 |
| | Tm = 146 | | | |

### Reference Example 20. Preparation of poly(I₂DTy-co-50%EGPLLAD7K carbonate).

In a 1 L 3-necked round-bottomed flask equipped with a mechanical stirrer, and a liquid addition device were placed 25 g (0.046 mol) of I₂DTy, 25 g (0.004 mol) of EGPLLAD, 14.8 g (0.19 mol) of pyridine, and 305 mL of DCM and stirred for 15 min to get a clear solution. Triphosgene (5.19 g, 0.053 mol of phosgene) was dissolved in 15 mL of DCM and the solution was introduced into the reaction flask over 2-3 hours. After the addition was complete, the reaction mixture was quenched with a mixture of 135 mL of THF and 15 mL of water. 350 mL of water was added to the reaction mixture and stirred for 5 min. After allowing the layers to separate, the top aqueous layer was removed and discarded. The washing was repeated with two additional 350 mL portions of DI water. The reaction mixture was then precipitated with 600 mL of IPA. The resulting gel was ground twice in a 4 L high speed blender. The precipitate obtained was isolated by filtration and dried in a vacuum oven at 80 °C. The polymer had a glass transition temperature (Tg) of 100 °C. ¹H NMR spectrum of the polymer was in agreement with the structure. Compression molding at 190 °C gave a uniform transparent film. Copolymers containing 45% and 60% of EGPLLAD were also prepared using similar procedures and characterized. The properties of the polymers are listed in the table below. Using similar procedures copolymers containing I₂DTE and polyglycolide-diols (PGAD) can be prepared by replacing PLLAD with PGAD in the above polymerization.

| % EGPLLAD | Tg, °C | σ, ksi | Modulus, ksi | Elongation, % |
|---|---|---|---|---|
| 45 | 62 and 106 | 8.2 | 275 | 17 |
| 50 | 62 and 106 | 8.0 | 247 | 106 |
| 60 | 60 | 7.9 | 257 | 188 |

### Reference Example 21. Preparation of poly(I₂DTy-co-50%DTy carbonate).

In a 1 L 3-necked round-bottomed flask equipped with a mechanical stirrer, and a liquid addition device were placed 25 g (0.046 mol) of I₂DTy, 25 g (0.087 mol) of DTy, 43 g (0.55 mol) of pyridine, and 305 mL of DCM and stirred for 15 min to get a clear solution. Triphosgene (14.2 g, 0.143 mol of phosgene) was dissolved in 43 mL of DCM and the solution was introduced into the reaction flask over 2-3 hours. After the addition was complete, the reaction mixture was quenched with a mixture of 135 mL of THF and 15 mL of water. 350 mL of water was added to the reaction mixture and stirred for 5 min. After allowing the layers to separate, the top aqueous layer was removed and discarded. The washing was repeated with two additional 350 mL portions of DI water. The reaction mixture was then precipitated with 600 mL of IPA. The resulting gel was ground twice in a 4 L high speed blender. The precipitate obtained was isolated by filtration and dried in a vacuum oven at 80 °C. The polymer had a glass transition temperature (Tg) of 68 °C. Compression molding at 170 °C gave a uniform transparent film which gave tensile modulus, tensile stress at yield, and elongation at break respectively of 195 ksi, 4.3 ksi, and 473%. Using similar procedure poly(I₂DTy-co-20%DTy carbonate was prepared.

### Reference Example 22. Synthesis of (4-(2-hydroxyethyl) 2,6,-diiodophenol).

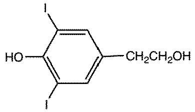

Iodination of tyrosol was carried out by adding 200 mL of KICl₂ solution (2M) to 27.6 g (0.2 mol) of tyrosol in 140 mL of 95% ethanol and stirring the resulting solution for 1 h. When treated with 400 mL of water, an oil separated which was stirred with 100 mL of 2% sodium thiosulfate solution for 2 h. The brown solid obtained was dissolved in ethanol and treated with charcoal and filtered. The pure diiodotyrosol (4-(2-hydroxyethyl) 2,6,-diiodophenol) was obtained in 65% yield and was characterized by hplc and NMR.

### Reference Example 23. Synthesis of 4-hydroxyphenethyl 3-(4-(4-hydroxyphenoxy)phenyl)-propanoate.

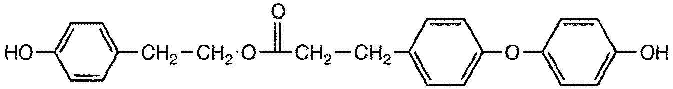

Into a 500 mL round bottomed flask fitted with an overhead stirrer, and a modified Dean-stark trap for solvents heavier than water are added 10 g (72 mmol) of tyrosol, 30 g (78 mmol) of desaminothyronine, 0.65 g (3.4 mmol) of 4-toluenesulfonic acid monohydrate, and 200 mL of 1.2-dichloroethane (DCE). A water-cooled reflux condenser is placed on top of the modified Dean-stark trap and the contents of the flask are heated to reflux while being stirred. The reaction is continued until approximately 1.4 mL of water collected in the modified Dean-stark trap above the DCE and the water collection essentially stopps (about 4 hours of reflux). The reaction mixture is cooled to room temperature and the crude product is dissolved in 100 mL of ethyl acetate and washed twice with 100 mL portions of 5% sodium bicarbonate solution. After drying over magnesium sulfate the organic layer is concentrated and precipitated with hexane. The resulting white crystalline solid is collected by filtration and dried in a vacuum oven at 25 °C. The product is characterized by elemental analysis, hplc, and ¹H NMR.

## Claims

1. A biocompatible polymer, comprising a recurring unit of formula: 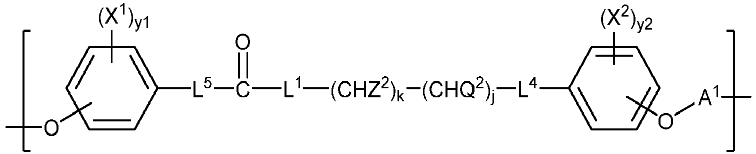 wherein:
j and k are each independently zero or an integer selected from 1 to 6, provided that j and k are not both 0;
X¹ and X² are each independently bromine (Br) or iodine (I), and y¹ and y² are each independently 0, 1, 2, 3, or 4;
L¹ is selected from the group consisting of oxygen (-O-), -R⁴-C(=O)-O-, and -O-R^{4a}-O-C(=O)- ; wherein R⁴ is selected from the group consisting of C₁-C₃₀ alkylene, C₂-C₃₀ alkenylene, C₂-C₃₀ alkynylene, C₁-C₃₀ heteroalkylene, C₂-C₃₀ heteroalkenylene, C₂-C₃₀ heteroalkynylene, C₆-C₃₀ arylene, C₇-C₃₀ alkylarylene, C₈-C₃₀ alkenylarylene, C₈-C₃₀ alkynylarylene, and C₂-C₃₀ heteroarylene; and R^{4a} is selected from the group consisting of C₁-C₃₀ alkylene, C₂-C₃₀ alkenylene, C₂-C₃₀ alkynylene, C₁-C₃₀ heteroalkylene, C₂-C₃₀ heteroalkenylene, C₂-C₃₀ heteroalkynylene, C₆-C₃₀ arylene, C₇-C₃₀ alkylarylene, C₈-C₃₀ alkenylarylene, C₈-C₃₀ alkynylarylene, and C₂-C₃₀ heteroarylene;
L⁴ is selected from the group consisting of a bond, and optionally substituted phenoxy (-C₆H₄-O-);
L⁵ is selected from the group consisting of a bond and -CH₂-;
Q² at each occurrence is independently hydrogen or halogen; or two adjacent Q² form a double bond when adjacent Qs are present; or Z² and an adjacent Q² form a double bond;
Z² is hydrogen, -N(R^{x})C(=O)R⁵, -N(R^{x})COOR⁶, -C(O)OR⁷ or -C(O)NR^{x}R^{y}, or Z² and an adjacent Q² form a double bond;
wherein R⁵, R⁶, R⁷, R^{x} and R^{y} are independently at each occurrence selected from the group consisting of hydrogen, alkyl, aryl, alkylaryl, arylalkyl, heteroalkyl, and heteroalkylaryl, wherein the non-hydrogen groups contain up to 30 carbon atoms, wherein the heteroalkyl group contains from 1 to 10 heteroatoms independently selected from O, N and S, and the heteroalkylaryl group contains from 1 to 3 heteroatoms independently selected from O, N and S; and
wherein A¹ is selected from the group consisting of: 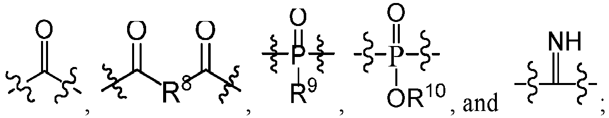
wherein R⁸ is selected from a bond, C₁-C₃₀ alkylene, C₂-C₃₀ alkenylene, C₂-C₃₀ alkynylene; C₁-C₃₀ heteroalkylene, C₂-C₃₀ heteroalkenylene, C₂-C₃₀ heteroalkynylene, C₇-C₃₀ heteroalkylarylene, C₈-C₃₀ heteroalkenylarylene, C₈-C₃₀ heteroalkynylarylene, C₇-C₃₀ alkylarylene, C₈-C₃₀ alkenylarylene, C₈-C₃₀ alkynylarylene, and C₂-C₃₀ heteroarylene; and
wherein R⁹ and R¹⁰ are each independently selected from H, C₁-C₃₀ alkyl, C₁-C₃₀ heteroalkyl, C₂-C₃₀ alkenyl, C₂-C₃₀ alkynyl, C₂-C₃₀ heteroalkenyl, and C₂-C₃₀ heteroalkynyl;
with the proviso that the biocompatible polymer is not poly(4'-hydroxyphenyethyl-2-(4-hydroxy-3,5-diiodophenyl)acetate carbonate).

2. A biocompatible polymer, comprising a recurring unit of formula: 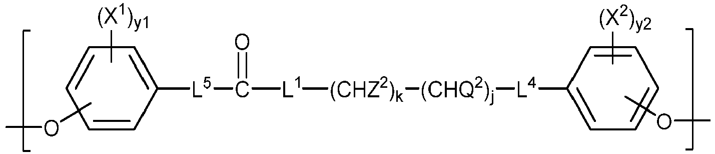 wherein:
j and k are each independently zero or an integer selected from 1 to 6, provided that j and k are not both 0;
X¹ and X² are each independently bromine (Br) or iodine (I), and y¹ and y² are each independently 0, 1, 2, 3, or 4;
L¹ is selected from the group consisting of oxygen (-O-), -R⁴-C(=O)-O-, and -O-R^{4a}-O-C(=O)- ; wherein R⁴ is selected from the group consisting of C₁-C₃₀ alkylene, C₂-C₃₀ alkenylene, C₂-C₃₀ alkynylene, C₁-C₃₀ heteroalkylene, C₂-C₃₀ heteroalkenylene, C₂-C₃₀ heteroalkynylene, C₆-C₃₀ arylene, C₇-C₃₀ alkylarylene, C₈-C₃₀ alkenylarylene, C₈-C₃₀ alkynylarylene, and C₂-C₃₀ heteroarylene; and R^{4a} is selected from the group consisting of C₁-C₃₀ alkylene, C₂-C₃₀ alkenylene, C₂-C₃₀ alkynylene, C₁-C₃₀ heteroalkylene, C₂-C₃₀ heteroalkenylene, C₂-C₃₀ heteroalkynylene, C₆-C₃₀ arylene, C₇-C₃₀ alkylarylene, C₈-C₃₀ alkenylarylene, C₈-C₃₀ alkynylarylene, and C₂-C₃₀ heteroarylene;
L⁴ is selected from the group consisting of a bond, and optionally substituted phenoxy (-C₆H₄-O-);
L⁵ is selected from the group consisting of a bond and -CH₂-;
Q² at each occurrence is independently hydrogen or halogen; or two adjacent Q² form a double bond when adjacent Qs are present; or Z² and an adjacent Q² form a double bond;
Z² is hydrogen, -N(R^{x})C(=O)R⁵, -N(R^{x})COOR⁶, -C(O)OR⁷ or -C(O)NR^{x}R^{y}, or Z² and an adjacent Q² form a double bond; and
wherein R⁵, R⁶, R⁷, R^{x} and R^{y} are independently at each occurrence selected from the group consisting of hydrogen, alkyl, aryl, alkylaryl, arylalkyl, heteroalkyl, and heteroalkylaryl, wherein the non-hydrogen groups contain up to 30 carbon atoms, wherein the heteroalkyl group contains from 1 to 10 heteroatoms independently selected from O, N and S, and the heteroalkylaryl group contains from 1 to 3 heteroatoms independently selected from O, N and S.

3. The biocompatible polymer of claim 1, wherein L5 is a bond, providing a recurring unit of formula:

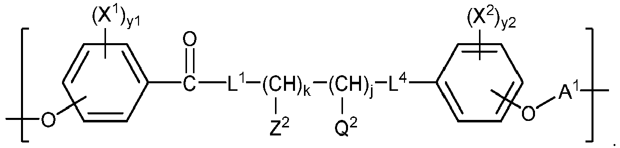

4. The biocompatible polymer of claim 2, wherein L5 is a bond, providing a recurring unit of formula:

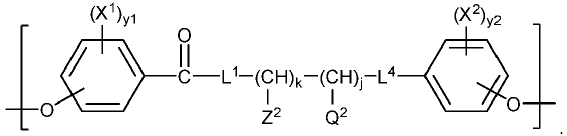

5. The biocompatible polymer of claim 3 or 4, wherein the phenyl groups of the recurring unit have the phenolic oxygens in the para-position.

6. The biocompatible polymer of any one of claims 3 to 5, wherein L⁴ is a bond, L¹ is oxygen, and Q² and Z² are both hydrogen.

7. The biocompatible polymer of claim 1, wherein j = k = 1, L¹ is oxygen, L⁴ and L⁵ are each a bond, and Q² and Z² are each hydrogen, providing a recurring unit of formula:

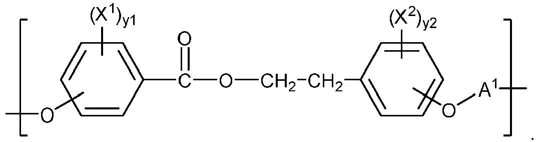

8. The biocompatible polymer of claim 2, wherein j = k = 1, L¹ is oxygen, L⁴ and L⁵ are each a bond, and Q² and Z² are each hydrogen, providing a recurring unit of formula:

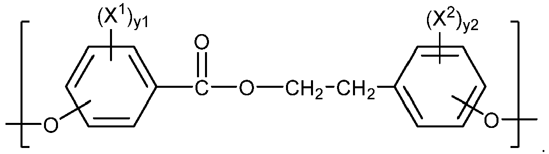

9. A biocompatible copolymer comprising the recurring unit of any one of the polymers of claims 1 to 8 and further comprising a) a tyrosol recurring unit of the formula: 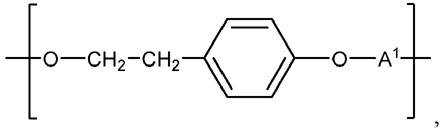
wherein A¹ is selected from the group consisting of: 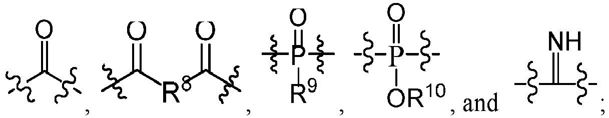
wherein R⁸ is selected from a bond, C₁-C₃₀ alkylene, C₂-C₃₀ alkenylene, C₂-C₃₀ alkynylene; C₁-C₃₀ heteroalkylene, C₂-C₃₀ heteroalkenylene, C₂-C₃₀ heteroalkynylene, C₇-C₃₀ heteroalkylarylene, C₈-C₃₀ heteroalkenylarylene, C₈-C₃₀ heteroalkynylarylene, C₇-C₃₀ alkylarylene, C₈-C₃₀ alkenylarylene, C₈-C₃₀ alkynylarylene, and C₂-C₃₀ heteroarylene; and
wherein R⁹ and R¹⁰ are each independently selected from H, C₁-C₃₀ alkyl, C₁-C₃₀ heteroalkyl, C₂-C₃₀ alkenyl, C₂-C₃₀ alkynyl, C₂-C₃₀ heteroalkenyl, and C₂-C₃₀ heteroalkynyl; or b) a recurring unit of the formula: 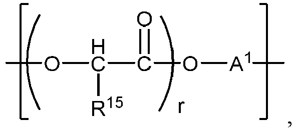
wherein R¹⁵ is H or CH₃, r is 1 to 300, and A¹ is selected from the group consisting of: 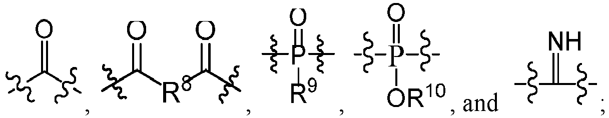
wherein R⁸ is selected from a bond, C₁-C₃₀ alkylene, C₂-C₃₀ alkenylene, C₂-C₃₀ alkynylene; C₁-C₃₀ heteroalkylene, C₂-C₃₀ heteroalkenylene, C₂-C₃₀ heteroalkynylene, C₇-C₃₀ heteroalkylarylene, C₈-C₃₀ heteroalkenylarylene, C₈-C₃₀ heteroalkynylarylene, C₇-C₃₀ alkylarylene, C₈-C₃₀ alkenylarylene, C₈-C₃₀ alkynylarylene, and C₂-C₃₀ heteroarylene; and
wherein R⁹ and R¹⁰ are each independently selected from H, C₁-C₃₀ alkyl, C₁-C₃₀ heteroalkyl, C₂-C₃₀ alkenyl, C₂-C₃₀ alkynyl, C₂-C₃₀ heteroalkenyl, and C₂-C₃₀ heteroalkynyl.

10. The biocompatible polymer of Claim 1, **characterized by** the formula: 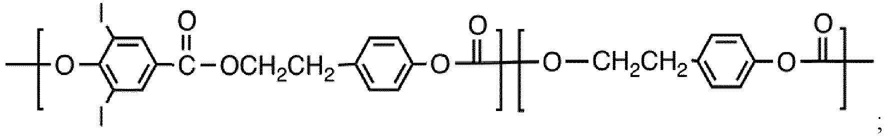 or
**characterized by** the formula: 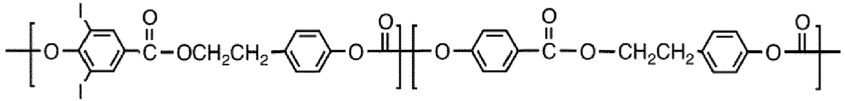 ; or
**characterized by** the formula: 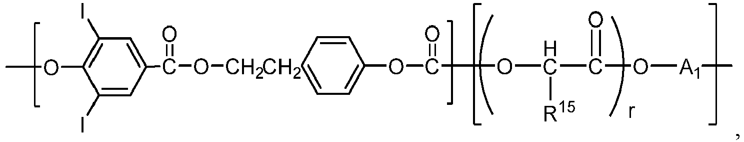 wherein R¹⁵ = H or CH₃, and r = 1 to 300.

11. The biocompatible polymer of claim 7, **characterized by** the formula:

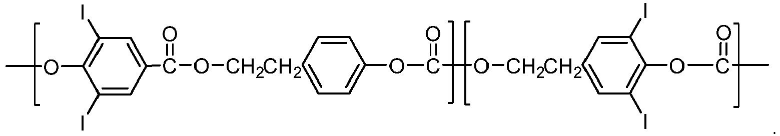

12. A diphenol compound having the formula: 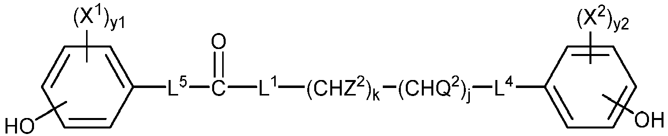 wherein:
j and k are each independently zero or an integer selected from 1 to 6, provided that j and k are not both 0;
X¹ and X² are each independently bromine (Br) or iodine (I), and y¹ and y² are each independently 0, 1, 2, 3, or 4;
L¹ is selected from the group consisting of oxygen (-O-), -R⁴-C(=O)-O-, and -O-R^{4a}-O-C(=O)- ; wherein R⁴ is selected from the group consisting of C₁-C₃₀ alkylene, C₂-C₃₀ alkenylene, C₂-C₃₀ alkynylene, C₁-C₃₀ heteroalkylene, C₂-C₃₀ heteroalkenylene, C₂-C₃₀ heteroalkynylene, C₆-C₃₀ arylene, C₇-C₃₀ alkylarylene, C₈-C₃₀ alkenylarylene, C₈-C₃₀ alkynylarylene, and C₂-C₃₀ heteroarylene; and R^{4a} is selected from the group consisting of C₁-C₃₀ alkylene, C₂-C₃₀ alkenylene, C₂-C₃₀ alkynylene, C₁-C₃₀ heteroalkylene, C₂-C₃₀ heteroalkenylene, C₂-C₃₀ heteroalkynylene, C₆-C₃₀ arylene, C₇-C₃₀ alkylarylene, C₈-C₃₀ alkenylarylene, C₈-C₃₀ alkynylarylene, and C₂-C₃₀ heteroarylene;
L⁴ is selected from the group consisting of a bond, and optionally substituted phenoxy (-C₆H₄-O-);
L⁵ is selected from the group consisting of a bond and -CH₂-;
Q² at each occurrence is independently hydrogen or halogen; or two adjacent Q² form a double bond when adjacent Qs are present; or Z² and an adjacent Q² form a double bond;
Z² is hydrogen, -N(R^{x})C(=O)R⁵, -N(R^{x})COOR⁶, -C(O)OR⁷ or -C(O)NR^{x}R^{y}, or Z² and an adjacent Q² form a double bond; and
wherein R⁵, R⁶, R⁷, R^{x} and R^{y} are independently at each occurrence selected from the group consisting of hydrogen, alkyl, aryl, alkylaryl, arylalkyl, heteroalkyl, and heteroalkylaryl, wherein the non-hydrogen groups contain up to 30 carbon atoms, wherein the heteroalkyl group contains from 1 to 10 heteroatoms independently selected from O, N and S, and the heteroalkylaryl group contains from 1 to 3 heteroatoms independently selected from O, N and S;
with the proviso that the diphenol compound is not

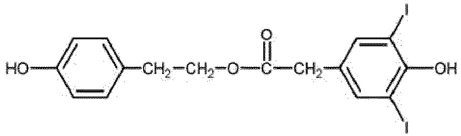

13. The diphenol compound of claim 12, wherein L⁵ is a bond; or wherein L⁴ and L⁵ are each a bond, L¹ is oxygen, Z² is hydrogen and Q² is hydrogen; or wherein L⁴ and L⁵ are each a bond, L¹ is oxygen, Z² is hydrogen, Q² is hydrogen and k = j = 1; or wherein L⁴ and L⁵ are each a bond, L¹ is oxygen, Q² and Z² are both hydrogen, k = j = 1, X¹ and X² are iodine, and at least one of y1 and y2 is non-zero.

14. A polymer composition comprising the biocompatible polymer of any one of claims 1-11.

15. A medical device, preferably a stent, comprising the biocompatible polymer of any one of claims 1-11.

## Patentansprüche

1. Biokompatibles Polymer, umfassend eine wiederkehrende Einheit der Formel: 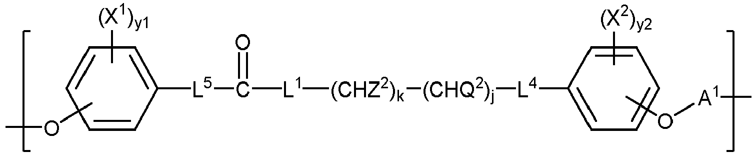 wobei:
j und k jeweils unabhängig null oder eine ganze Zahl sind, die aus 1 bis 6 ausgewählt ist, mit der Maßgabe, dass j und k nicht jeweils 0 sind;
X¹ und X² jeweils unabhängig Brom (Br) oder lod (I) sind und y¹ und y² jeweils unabhängig 0, 1, 2, 3 oder 4 sind;
L¹ aus der Gruppe ausgewählt ist, die aus Sauerstoff (-O-), -R⁴-C(=O)-O- und -O-R^{4a}-O-C(=O)- besteht; wobei R⁴ aus der Gruppe ausgewählt ist, die aus C₁-C₃₀-Alkylen, C₂-C₃₀-Alkenylen, C₂-C₃₀-Alkynylen, C₁-C₃₀-Heteroalkylen, C₂-C₃₀-Heteroalkenylen, C₂-C₃₀-Heteroalkynylen, C₆-C₃₀-Arylen, C₇-C₃₀-Alkylarylen, C₈-C₃₀-Alkenylarylen, C₈-C₃₀-Alkynylarylen und C₂-C₃₀-Heteroarylen besteht; und R^{4a} aus der Gruppe ausgewählt ist, die aus C₁-C₃₀-Alkylen, C₂-C₃₀-Alkenylen, C₂-C₃₀-Alkynylen, C₁-C₃₀-Heteroalkylen, C₂-C₃₀-Heteroalkenylen, C₂-C₃₀-Heteroalkynylen, C₆-C₃₀-Arylen, C₇-C₃₀-Alkylarylen, C₈-C₃₀-Alkenylarylen, C₈-C₃₀-Alkynylarylen und C₂-C₃₀-Heteroarylen besteht;
L⁴ aus der Gruppe ausgewählt ist, die aus einer Bindung und wahlweise substituiertem Phenoxy (-C₆H₄-O-) besteht;
L⁵ aus der Gruppe ausgewählt ist, die aus einer Bindung und -CH₂- besteht;
Q² bei jedem Vorkommen unabhängig Wasserstoff oder Halogen ist; oder zwei angrenzende Q² eine Doppelbindung bilden, wenn angrenzende Q vorhanden sind; oder Z² und ein angrenzendes Q² eine Doppelbindung bilden;
Z² Wasserstoff, -N(R^{x})C(=O)R⁵, -N(R^{x})COOR⁶, -C(O)OR⁷ oder -C(O)NR^{x}R^{y} ist oder Z² und ein angrenzendes Q² eine Doppelbindung bilden;
wobei R⁵, R⁶, R⁷, R^{x} und R^{y} bei jedem Vorkommen unabhängig aus der Gruppe ausgewählt sind, die aus Wasserstoff, Alkyl, Aryl, Alkylaryl, Arylalkyl, Heteroalkyl und Heteroalkylaryl besteht, wobei die Nicht-Wasserstoffgruppen bis zu 30 Kohlenstoffatome enthalten, wobei die Heteroalkylgruppe 1 bis 10 Heteroatome enthält, die unabhängig aus O, N und S ausgewählt sind, und
die Heteroalkylarylgruppe 1 bis 3 Heteroatome enthält, die unabhängig aus O, N und S ausgewählt sind; und
wobei A¹ aus der Gruppe ausgewählt ist, die besteht aus: 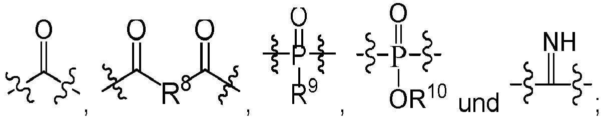
wobei R⁸ aus einer Bindung, C₁-C₃₀-Alkylen, C₂-C₃₀-Alkenylen, C₂-C₃₀₋Alkynylen; C₁-C₃₀-Heteroalkylen, C₂-C₃₀-Heteroalkenylen, C₂-C₃₀₋Heteroalkynylen, C₇-C₃₀-Heteroalkylarylen, C₈-C₃₀-Heteroalkenylarylen, C₈-C₃₀-Heteroalkynylarylen, C₇-C₃₀₋Alkylarylen, C₈-C₃₀-Alkenylarylen, C₈-C₃₀-Alkynylarylen und C₂-C₃₀-Heteroarylen ausgewählt ist; und
wobei R⁹ und R¹⁰ jeweils unabhängig aus H, C₁-C₃₀-Alkyl, C₁-C₃₀-Heteroalkyl, C₂-C₃₀-Alkenyl, C₂-C₃₀-Alkynyl, C₂-C₃₀-Heteroalkenyl und C₂-C₃₀-Heteroalkynyl ausgewählt sind;
mit der Maßgabe, dass das biokompatible Polymer nicht Poly(4'-hydroxyphenyethyl-2-(4-hydroxy-3,5-diiodphenyl)acetatcarbonat) ist.

2. Biokompatibles Polymer, umfassend eine sich wiederholende Einheit der Formel: 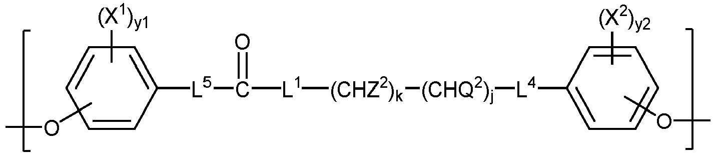 wobei:
j und k jeweils unabhängig null oder eine ganze Zahl sind, die aus 1 bis 6 ausgewählt ist, mit der Maßgabe, dass j und k nicht jeweils 0 sind;
X¹ und X² jeweils unabhängig Brom (Br) oder lod (I) sind und y¹ und y² jeweils unabhängig 0, 1, 2, 3 oder 4 sind;
L¹ aus der Gruppe ausgewählt ist, die aus Sauerstoff (-O-), -R⁴-C(=O)-O- und -O-R^{4a}-O-C(=O)- besteht; wobei R⁴ aus der Gruppe ausgewählt ist, die aus C₁-C₃₀-Alkylen, C₂-C₃₀-Alkenylen, C₂-C₃₀-Alkynylen, C₁-C₃₀-Heteroalkylen, C₂-C₃₀-Heteroalkenylen, C₂-C₃₀-Heteroalkynylen, C₆-C₃₀-Arylen, C₇-C₃₀-Alkylarylen, C₈-C₃₀-Alkenylarylen, C₈-C₃₀-Alkynylarylen und C₂-C₃₀-Heteroarylen besteht; und R^{4a} aus der Gruppe ausgewählt ist, die aus C₁-C₃₀-Alkylen, C₂-C₃₀-Alkenylen, C₂-C₃₀-Alkynylen, C₁-C₃₀-Heteroalkylen, C₂-C₃₀-Heteroalkenylen, C₂-C₃₀-Heteroalkynylen, C₆-C₃₀-Arylen, C₇-C₃₀-Alkylarylen, C₈-C₃₀-Alkenylarylen, C₈-C₃₀-Alkynylarylen und C₂-C₃₀-Heteroarylen besteht;
L⁴ aus der Gruppe ausgewählt ist, die aus einer Bindung und wahlweise substituiertem Phenoxy (-C₆H₄-O-) besteht;
L⁵ aus der Gruppe ausgewählt ist, die aus einer Bindung und -CH₂- besteht;
Q² bei jedem Vorkommen unabhängig Wasserstoff oder Halogen ist; oder zwei angrenzende Q² eine Doppelbindung bilden, wenn angrenzende Q vorhanden sind; oder Z² und ein angrenzendes Q² eine Doppelbindung bilden;
Z² Wasserstoff, -N(R^{x})C(=O)R⁵, -N(R^{x})COOR⁶, -C(O)OR⁷ oder -C(O)NR^{x}R^{y} ist oder Z² und ein angrenzendes Q² eine Doppelbindung bilden; und
wobei R⁵, R⁶, R⁷, R^{x} und R^{y} bei jedem Vorkommen unabhängig aus der Gruppe ausgewählt sind, die aus Wasserstoff, Alkyl, Aryl, Alkylaryl, Arylalkyl, Heteroalkyl und Heteroalkylaryl besteht, wobei die Nicht-Wasserstoffgruppen bis zu 30 Kohlenstoffatome enthalten, wobei die Heteroalkylgruppe 1 bis 10 Heteroatome enthält, die unabhängig aus O, N und S ausgewählt sind, und die Heteroalkylarylgruppe 1 bis 3 Heteroatome enthält, die unabhängig aus O, N und S ausgewählt sind.

3. Biokompatibles Polymer nach Anspruch 1, wobei L5 eine Bindung ist, die eine wiederkehrende Einheit der Formel: 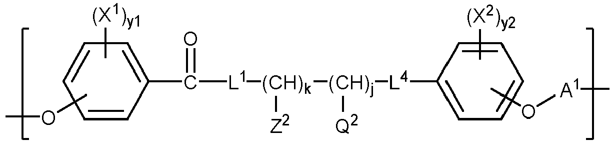 bereitstellt.

4. Biokompatibles Polymer nach Anspruch 2, wobei L5 eine Bindung ist, die eine wiederkehrende Einheit der Formel: 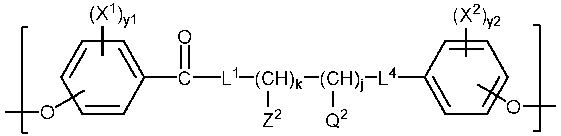 bereitstellt.

5. Biokompatibles Polyner nach Anspruch 3 oder 4, wobei die Phenylgruppen der wiederkehrenden Einheit die Phenolsauerstoffe in der para-Position aufweisen.

6. Biokompatibles Polymer nach einem der Ansprüche 3 bis 5, wobei L⁴ eine Bindung ist, L¹ Sauerstoff ist und Q² und Z² jeweils Wasserstoff sind.

7. Biokompatibles Polymer nach Anspruch 1, wobei j = k = 1, L¹ Sauerstoff ist, L⁴ und L⁵ jeweils eine Bindung sind und Q² und Z² jeweils Wasserstoff sind, wobei eine wiederkehrende Einheit der Formel: 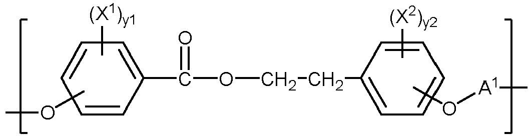 bereitgestellt wird.

8. Biokompatibles Polymer nach Anspruch 2, wobei j = k = 1, L¹ Sauerstoff ist, L⁴ und L⁵ jeweils eine Bindung sind und Q² und Z² jeweils Wasserstoff sind, wobei eine wiederkehrende Einheit der Formel: 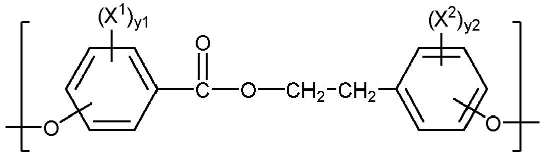 bereitgestellt wird.

9. Biokompatibles Copolymer, das die wiederkehrende Einheit eines der Polymere nach Anspruch 1 bis 8 umfasst und das ferner a) eine wiederkehrende Tyrosoleinheit der Formel: 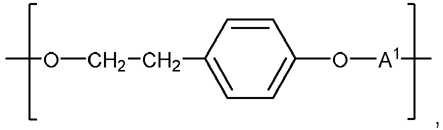
wobei A¹ aus der Gruppe ausgewählt ist, die besteht aus: 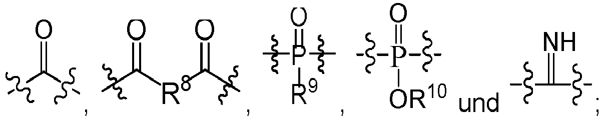
wobei R⁸ aus einer Bindung, C₁-C₃₀-Alkylen, C₂-C₃₀-Alkenylen, C₂-C₃₀₋Alkynylen; C₁-C₃₀-Heteroalkylen, C₂-C₃₀-Heteroalkenylen, C₂-C₃₀₋Heteroalkynylen, C₇-C₃₀-Heteroalkylarylen, C₈-C₃₀-Heteroalkenylarylen, C₈-C₃₀-Heteroalkynylarylen, C₇-C₃₀₋Alkylarylen, C₈-C₃₀-Alkenylarylen, C₈-C₃₀-Alkynylarylen und C₂-C₃₀-Heteroarylen ausgewählt ist; und
wobei R⁹ und R¹⁰ jeweils unabhängig aus H, C₁-C₃₀-Alkyl, C₁-C₃₀-Heteroalkyl, C₂-C₃₀-Alkenyl, C₂-C₃₀-Alkynyl, C₂-C₃₀-Heteroalkenyl und C₂-C₃₀-Heteroalkynyl ausgewählt sind; oder b) eine wiederkehrende Einheit der Formel: 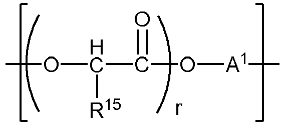
umfasst, wobei R¹⁵ H oder CH₃ ist, r 1 bis 300 ist und A¹ aus der Gruppe ausgewählt ist, die besteht aus: 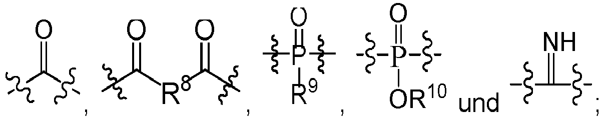
wobei R⁸ aus einer Bindung, C₁-C₃₀-Alkylen, C₂-C₃₀-Alkenylen, C₂-C₃₀₋Alkynylen; C₁-C₃₀-Heteroalkylen, C₂-C₃₀-Heteroalkenylen, C₂-C₃₀₋Heteroalkynylen, C₇-C₃₀-Heteroalkylarylen, C₈-C₃₀-Heteroalkenylarylen, C₈-C₃₀-Heteroalkynylarylen, C₇-C₃₀₋Alkylarylen, C₈-C₃₀₋Alkenylarylen, C₈-C₃₀₋Alkynylarylen und C₂-C₃₀-Heteroarylen ausgewählt ist; und
wobei R⁹ und R¹⁰ jeweils unabhängig aus H, C₁-C₃₀-Alkyl, C₁-C₃₀-Heteroalkyl, C₂-C₃₀-Alkenyl, C₂-C₃₀-Alkynyl, C₂-C₃₀-Heteroalkenyl und C₂-C₃₀-Heteroalkynyl ausgewählt sind.

10. Biokompatibles Polymer nach Anspruch 1, **gekennzeichnet durch** die Formel: 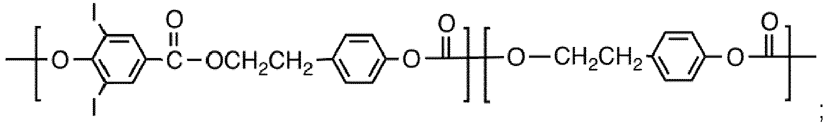 oder
**gekennzeichnet durch** die Formel: 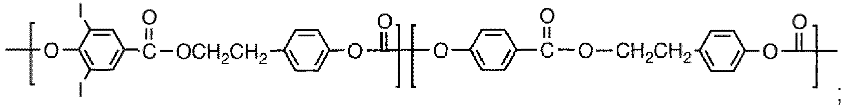 oder
**gekennzeichnet durch** die Formel: 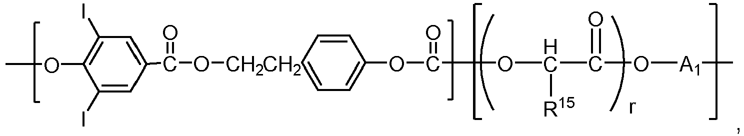
wobei R¹⁵ = H oder CH₃ und r = 1 bis 300.

11. Biokompatibles Polymer nach Anspruch 7, **gekennzeichnet durch** die Formel:

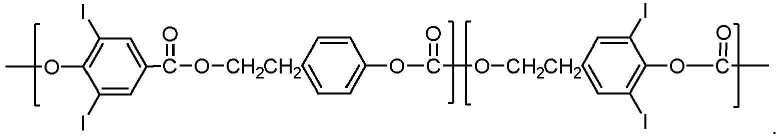

12. Diphenolverbindung mit der Formel: 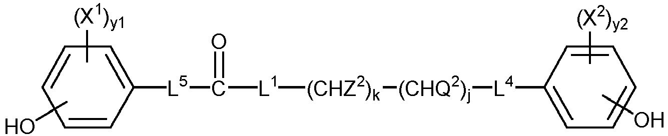 wobei:
j und k jeweils unabhängig null oder eine ganze Zahl sind, die aus 1 bis 6 ausgewählt ist, mit der Maßgabe, dass j und k nicht jeweils 0 sind;
X¹ und X² jeweils unabhängig Brom (Br) oder lod (I) sind und y¹ und y² jeweils unabhängig 0, 1, 2, 3 oder 4 sind;
L¹ aus der Gruppe ausgewählt ist, die aus Sauerstoff (-O-), -R⁴-C(=O)-O- und -O-R^{4a}-O-C(=O)- besteht; wobei R⁴ aus der Gruppe ausgewählt ist, die aus C₁-C₃₀-Alkylen, C₂-C₃₀-Alkenylen, C₂-C₃₀-Alkynylen, C₁-C₃₀-Heteroalkylen, C₂-C₃₀-Heteroalkenylen, C₂-C₃₀-Heteroalkynylen, C₆-C₃₀-Arylen, C₇-C₃₀-Alkylarylen, C₈-C₃₀-Alkenylarylen, C₈-C₃₀-Alkynylarylen und C₂-C₃₀-Heteroarylen besteht; und R^{4a} aus der Gruppe ausgewählt ist, die aus C₁-C₃₀-Alkylen, C₂-C₃₀-Alkenylen, C₂-C₃₀-Alkynylen, C₁-C₃₀-Heteroalkylen, C₂-C₃₀-Heteroalkenylen, C₂-C₃₀-Heteroalkynylen, C₆-C₃₀-Arylen, C₇-C₃₀-Alkylarylen, C₈-C₃₀-Alkenylarylen, C₈-C₃₀-Alkynylarylen und C₂-C₃₀-Heteroarylen besteht;
L⁴ aus der Gruppe ausgewählt ist, die aus einer Bindung und wahlweise substituiertem Phenoxy (-C₆H₄-O-) besteht;
L⁵ aus der Gruppe ausgewählt ist, die aus einer Bindung und -CH₂- besteht;
Q² bei jedem Vorkommen unabhängig Wasserstoff oder Halogen ist; oder zwei angrenzende Q² eine Doppelbindung bilden, wenn angrenzende Q vorhanden sind; oder Z² und ein angrenzendes Q² eine Doppelbindung bilden;
Z² Wasserstoff, -N(R^{x})C(=O)R⁵, -N(R^{x})COOR⁶, -C(O)OR⁷ oder -C(O)NR^{x}R^{y} ist oder Z² und ein angrenzendes Q² eine Doppelbindung bilden; und
wobei R⁵, R⁶, R⁷, R^{x} und R^{y} bei jedem Vorkommen unabhängig aus der Gruppe ausgewählt sind, die aus Wasserstoff, Alkyl, Aryl, Alkylaryl, Arylalkyl,
Heteroalkyl und Heteroalkylaryl besteht, wobei die Nicht-Wasserstoffgruppen bis zu 30 Kohlenstoffatome enthalten, wobei die Heteroalkylgruppe 1 bis 10 Heteroatome enthält, die unabhängig aus O, N und S ausgewählt sind, und die Heteroalkylarylgruppe 1 bis 3 Heteroatome enthält, die unabhängig aus O, N und S ausgewählt sind;
mit der Maßgabe, dass die Diphenolverbindung nicht 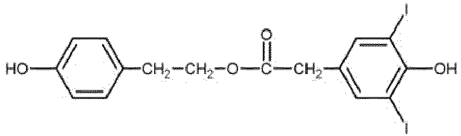 ist.

13. Diphenolverbindung nach Anspruch 12, wobei L⁵ eine Bindung ist; oder wobei L⁴ und L⁵ jeweils eine Bindung sind, L¹ Sauerstoff ist, Z² Wasserstoff ist und Q² Wasserstoff ist; oder wobei L⁴ und L⁵ jeweils eine Bindung sind, L¹ Sauerstoff ist, Z² Wasserstoff ist, Q² Wasserstoff ist und k = j = 1; oder wobei L⁴ und L⁵ jeweils eine Bindung sind, L¹ Sauerstoff ist, Q² und Z² jeweils Wasserstoff sind, k = j = 1, X¹ und X² lod sind und zumindest eines von y1 und y2 nicht null ist.

14. Polymerzusammensetzung, die das biokompatible Polymer nach einem der Ansprüche 1 bis 11 umfasst.

15. Medizinische Vorrichtung, vorzugsweise ein Stent, die das biokompatible Polymer nach einem der Ansprüche 1 bis 11 umfasst.

## Revendications

1. Polymère biocompatible, comprenant une unité récurrente de formule : 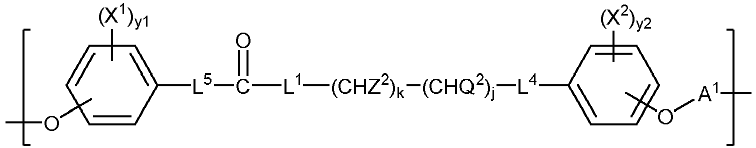 dans laquelle :
j et k sont chacun indépendamment zéro ou un nombre entier choisi de 1 à 6, à condition que j et k ne soient pas tous les deux 0 ;
X¹ et X² sont chacun indépendamment brome (Br) ou iode (I), et y¹ et y² sont chacun indépendamment 0, 1, 2, 3 ou 4 ;
L¹ est choisi dans le groupe consistant en oxygène (-O-), -R⁴-C(=O)-O-, et -O-R^{4a}-O-C(=O)- ; dans lequel R⁴ est choisi dans le groupe consistant en alkylène en C₁-C₃₀, alcénylène en C₂-C₃₀, alcynylène en C₂-C₃₀, hétéroalkylène en C₁-C₃₀, hétéroalcénylène en C₂-C₃₀, hétéroalcynylène en C₂-C₃₀, arylène en C₆-C₃₀, alkylarylène en C₇-C₃₀, alcénylarylène en C₈-C₃₀, alcynylarylène en C₈-C₃₀, et hétéroarylène en C₂-C₃₀, et R^{4a} est choisi dans le groupe consistant en alkylène en C₁-C₃₀, alcénylène en C₂-C₃₀, alcynylène en C₂-C₃₀, hétéroalkylène en C₁-C₃₀, hétéroalcénylène en C₂-C₃₀, hétéroalcynylène en C₂-C₃₀, arylène en C₆-C₃₀, alkylarylène en C₇-C₃₀, alcénylarylène en C₈-C₃₀, alcynylarylène en C₈-C₃₀, et hétéroarylène en C₂-C₃₀ ;
L⁴ est choisi dans le groupe constitué d'une liaison et d'un phénoxy éventuellement substitué (-C₆H₄-O-) ;
L⁵ est choisi dans le groupe consistant en une liaison et -CH₂- ;
Q² à chaque occurrence est indépendamment un hydrogène ou un halogène ; ou deux Q² adjacents forment une double liaison lorsque des Q adjacents sont présents ; ou Z² et un Q² adjacent forment une double liaison ;
Z² est hydrogène, -N(R^{x})C(=O)R⁵, -N(R^{x})COOR⁶, -C(O)OR⁷ ou -C(O)NR^{x}R^{y}, ou Z² et un Q² adjacent forment une double liaison ;
R⁵, R⁶, R⁷, R^{x} et R^{y} étant indépendamment à chaque occurrence choisis dans le groupe consistant en hydrogène, alkyle, aryle, alkylaryle, arylalkyle, hétéroalkyle et hétéroalkylaryle, où les groupes non hydrogène contiennent jusqu'à 30 atomes de carbone, où le groupe d'hétéroalkyle contient de 1 à 10 hétéroatomes choisis indépendamment parmi O, N et S, et le groupe d'hétéroalkylaryle contient de 1 à 3 hétéroatomes choisis indépendamment parmi O, N et S ; et ;
dans laquelle A¹ est choisi dans le groupe consistant en : 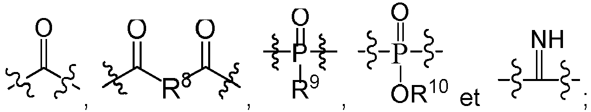
dans lequel R⁸ est choisi parmi une liaison, alkylène en C₁-C₃₀, alcénylène en C₂-C₃₀, alcynylène en C₂-C₃₀ ; hétéroalkylène en C₁-C₃₀, hétéroalcénylène en C₂-C₃₀, hétéroalcynylène en C₂-C₃₀, hétéroalkylarylène en C₇-C₃₀, hétéroalcénylarylène en C₈-C₃₀, hétéroalcynylarylène en C₈-C₃₀, alkylarylène en C₇-C₃₀, alcénylarylène en C₈-C₃₀, alcynylarylène en C₈-C₃₀, et hétéroarylène en C₂-C₃₀ ; et
dans lequel R9 et R10 sont chacun indépendamment choisis parmi H, alkyle en C₁-C₃₀, hétéroalkyle en C₁-C₃₀, alcényle en C₂-C₃₀, alcynyle en C₂-C₃₀, hétéroalcényle en C₂-C₃₀ et un hétéroalcynyle en C₂-C₃₀ ;
à condition que le polymère biocompatible ne soit pas un carbonate de poly(4'-hydroxyphényéthyl-2-(4-hydroxy-3,5-diiodophényl)acétate).

2. Polymère biocompatible, comprenant une unité récurrente de formule : 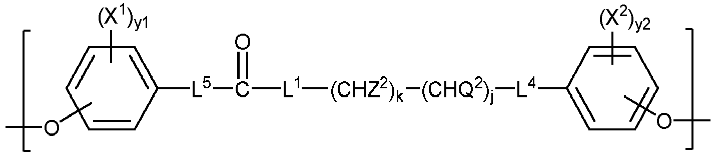 dans laquelle :
j et k sont chacun indépendamment zéro ou un nombre entier choisi de 1 à 6, à condition que j et k ne soient pas tous les deux 0 ;
X¹ et X² sont chacun indépendamment brome (Br) ou iode (I), et y¹ et y² sont chacun indépendamment 0, 1, 2, 3 ou 4 ;
L¹ est choisi dans le groupe consistant en oxygène (-O-), -R⁴-C(=O)-O-, et -O-R^{4a}-O-C(=O)- ; dans lequel R⁴ est choisi dans le groupe consistant en alkylène en C₁-C₃₀, alcénylène en C₂-C₃₀, alcynylène en C₂-C₃₀, hétéroalkylène en C₁-C₃₀, hétéroalcénylène en C₂-C₃₀, hétéroalcynylène en C₂-C₃₀, arylène en C₆-C₃₀, alkylarylène en C₇-C₃₀, alcénylarylène en C₈-C₃₀, alcynylarylène en C₈-C₃₀ et hétéroarylène en C₂-C₃₀, et R^{4a} est choisi dans le groupe consistant en alkylène en C₁-C₃₀, alcénylène en C₂-C₃₀, alcynylène en C₂-C₃₀, hétéroalkylène en C₁-C₃₀, hétéroalcénylène en C₂-C₃₀, hétéroalcynylène en C₂-C₃₀, arylène en C₆-C₃₀, alkylarylène en C₇-C₃₀, alcénylarylène en C₈-C₃₀, alcynylarylène en C₈-C₃₀, et hétéroarylène en C₂-C₃₀ ;
L⁴ est choisi dans le groupe constitué d'une liaison et d'un phénoxy éventuellement substitué (-C₆H₄-O-) ;
L⁵ est choisi dans le groupe consistant en une liaison et -CH₂- ;
Q² à chaque occurrence est indépendamment un hydrogène ou un halogène ; ou deux Q² adjacents forment une double liaison lorsque des Q adjacents sont présents ; ou Z² et un Q² adjacent forment une double liaison ;
Z² est hydrogène, -N(R^{x})C(=O)R⁵, -N(R^{x})COOR⁶, -C(O)OR⁷ ou -C(O)NR^{x}R^{y}, ou Z² et un Q² adjacent forment une double liaison ;
R⁵, R⁶, R⁷, R^{x} et R^{y} étant indépendamment à chaque occurrence choisis dans le groupe consistant en hydrogène, alkyle, aryle, alkylaryle, arylalkyle, hétéroalkyle et hétéroalkylaryle, où les groupes non hydrogène contiennent jusqu'à 30 atomes de carbone, où le groupe d'hétéroalkyle contient de 1 à 10 hétéroatomes choisis indépendamment parmi O, N et S, et le groupe d'hétéroalkylaryle contient de 1 à 3 hétéroatomes choisis indépendamment parmi O, N et S.

3. Polymère biocompatible selon la revendication 1, dans lequel L5 est une liaison, fournissant une unité récurrente de formule :

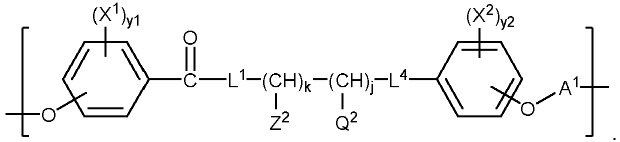

4. Polymère biocompatible selon la revendication 2, dans lequel L5 est une liaison, fournissant une unité récurrente de formule :

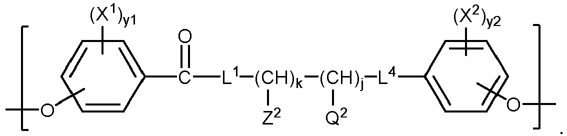

5. Polymère biocompatible selon la revendication 3 ou 4, dans lequel les groupes phényle de l'unité récurrente ont les oxygènes phénoliques en position para.

6. Polymère biocompatible selon l'une quelconque des revendications 3 à 5, dans lequel L⁴ est une liaison, L¹ est l'oxygène et Q² et Z² sont tous deux l'hydrogène.

7. Polymère biocompatible selon la revendication 1, dans lequel j = k = 1, L¹ est l'oxygène, L⁴ et L⁵ sont chacun une liaison, et Q² et Z² sont chacun l'hydrogène, fournissant une unité récurrente de formule :

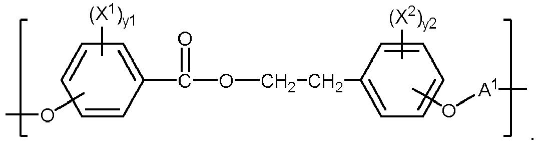

8. Polymère biocompatible selon la revendication 2, dans lequel j = k = 1, L¹ est l'oxygène, L⁴ et L⁵ sont chacun une liaison, et Q² et Z² sont chacun l'hydrogène, fournissant une unité récurrente de formule :

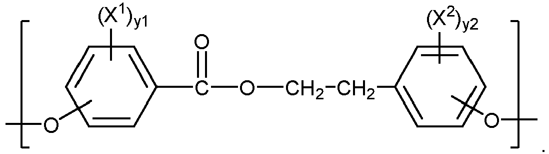

9. Copolymère biocompatible comprenant l'unité récurrente de l'un quelconque des polymères des revendications 1 à 8 et comprenant en outre a) une unité récurrente de tyrosol de la formule : 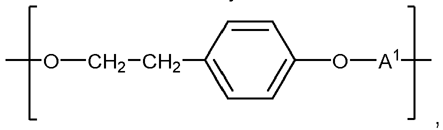
dans laquelle A¹ est choisi dans le groupe consistant en : 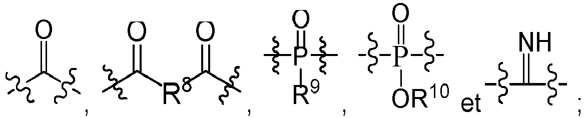
dans lequel R⁸ est choisi parmi une liaison, alkylène en C₁-C₃₀, alcénylène en C₂-C₃₀, alcynylène en C₂-C₃₀ ; hétéroalkylène en C₁-C₃₀, hétéroalcénylène en C₂-C₃₀, hétéroalcynylène en C₂-C₃₀, hétéroalkylarylène en C₇-C₃₀, hétéroalcénylarylène en C₈-C₃₀, hétéroalcynylarylène en C₈-C₃₀, alkylarylène en C₇-C₃₀, alcénylarylène en C₈-C₃₀, alcynylarylène en C₈-C₃₀, et hétéroarylène en C₂-C₃₀ ; et
dans lequel R⁹ et R¹⁰ sont chacun indépendamment choisis parmi H, alkyle en C₁-C₃₀, hétéroalkyle en C₁-C₃₀, alcényle en C₂-C₃₀, alcynyle en C₂-C₃₀, hétéroalcényle en C₂-C₃₀ et hétéroalcynyle en C₂-C₃₀ ; ou b) une unité récurrente de la formule : 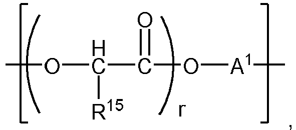
dans laquelle R¹⁵ est H ou CH₃, r est 1 à 300, et A¹ est choisi du groupe consistant en : 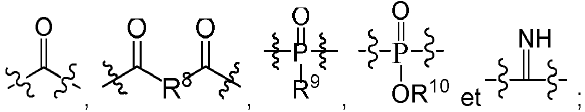
dans lequel R⁸ est choisi parmi une liaison, alkylène en C₁-C₃₀, alcénylène en C₂-C₃₀, alcynylène en C₂-C₃₀ ; hétéroalkylène en C₁-C₃₀, hétéroalcénylène en C₂-C₃₀, hétéroalcynylène en C₂-C₃₀, hétéroalkylarylène en C₇-C₃₀, hétéroalcénylarylène en C₈-C₃₀, hétéroalcynylarylène en C₈-C₃₀, alkylarylène en C₇-C₃₀, alcénylarylène en C₈-C₃₀ , alcynylarylène en C₈-C₃₀ , et hétéroarylène en C₂-C₃₀ ; et
dans lequel R⁹ et R¹⁰ sont chacun indépendamment choisis parmi H, alkyle en C₁-C₃₀, hétéroalkyle en C₁-C₃₀, alcényle en C₂-C₃₀, alcynyle en C₂-C₃₀, hétéroalcényle en C₂-C₃₀ et hétéroalcynyle en C₂-C₃₀.

10. Polymère biocompatible selon la revendication 1, **caractérisé par** la formule : 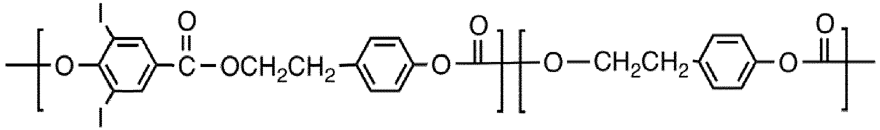 ; ou
**caractérisé par** la formule : 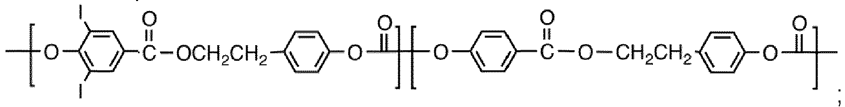 ou
**caractérisé par** la formule : 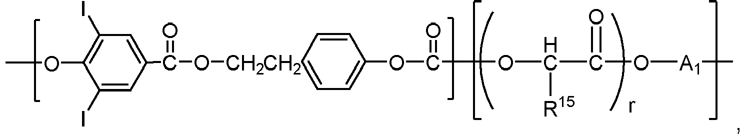
dans laquelle R¹⁵ = H ou CH₃, et r = 1 à 300.

11. Polymère biocompatible selon la revendication 7, **caractérisé par** la formule :

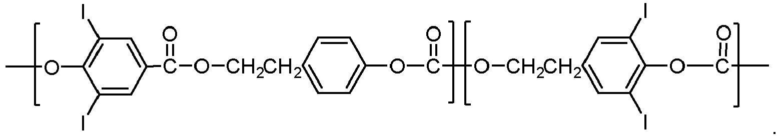

12. Composé diphénol avec la formule : 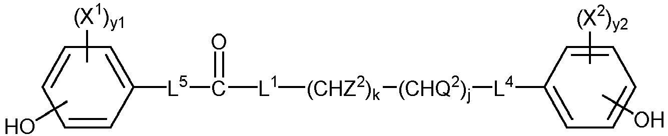 dans laquelle :
j et k sont chacun indépendamment zéro ou un nombre entier choisi de 1 à 6, à condition que j et k ne soient pas tous les deux 0 ;
X¹ et X² sont chacun indépendamment brome (Br) ou iode (I), et y¹ et y² sont chacun indépendamment 0, 1, 2, 3 ou 4 ;
L¹ est choisi dans le groupe consistant en oxygène (-O-), -R⁴-C(=O)-O-, et -O-R^{4a}-O-C(=O)- ; dans lequel R⁴ est choisi dans le groupe consistant en alkylène en C₁-C₃₀, alcénylène en C₂-C₃₀, alcynylène en C₂-C₃₀, hétéroalkylène en C₁-C₃₀, hétéroalcénylène en C₂-C₃₀, hétéroalcynylène en C₂-C₃₀, arylène en C₆-C₃₀, alkylarylène en C₇-C₃₀, alcénylarylène en C₈-C₃₀, alcynylarylène en C₈-C₃₀, et hétéroarylène en C₂-C₃₀, et R^{4a} est choisi dans le groupe consistant en alkylène en C₁-C₃₀, alcénylène en C₂-C₃₀, alcynylène en C₂-C₃₀, hétéroalkylène en C₁-C₃₀, hétéroalcénylène en C₂-C₃₀, hétéroalcynylène en C₂-C₃₀, arylène en C₆-C₃₀, alkylarylène en C₇-C₃₀, alcénylarylène en C₈-C₃₀, alcynylarylène en C₈-C₃₀, et hétéroarylène en C₂-C₃₀ ;
L⁴ est choisi dans le groupe constitué d'une liaison et d'un phénoxy éventuellement substitué (-C₆H₄-O-) ;
L⁵ est choisi dans le groupe consistant en une liaison et -CH₂- ;
Q² à chaque occurrence est indépendamment un hydrogène ou un halogène ; ou deux Q² adjacents forment une double liaison lorsque des Q adjacents sont présents ; ou Z² et un Q² adjacent forment une double liaison ;
Z² est hydrogène, -N(R^{x})C(=O)R⁵, -N(R^{x})COOR⁶, -C(O)OR⁷ ou -C(O)NR^{x}R^{y}, ou Z² et un Q² adjacent forment une double liaison ;
R⁵, R⁶, R⁷, R^{x} et R^{y} étant indépendamment à chaque occurrence choisis dans le groupe consistant en hydrogène, alkyle, aryle, alkylaryle, arylalkyle, hétéroalkyle et hétéroalkylaryle, où les groupes non hydrogène contiennent jusqu'à 30 atomes de carbone, où le groupe d'hétéroalkyle contient de 1 à 10 hétéroatomes choisis indépendamment parmi O, N et S, et le groupe d'hétéroalkylaryle contient de 1 à 3 hétéroatomes choisis indépendamment parmi O, N et S ;
à condition que le composé diphénol ne soit pas

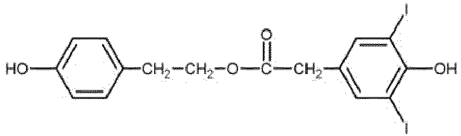

13. Composé diphénol selon la revendication 12, dans lequel L⁵ est une liaison ; ou dans lequel L⁴ et L⁵ sont chacun une liaison, L¹ est l'oxygène, Z² est l'hydrogène et Q² est l'hydrogène ; ou dans lequel L⁴ et L⁵ sont chacun une liaison, L¹ est l'oxygène, Z² est l'hydrogène, Q² est l'hydrogène, et k = j = 1; ou dans lequel L⁴ et L⁵ sont chacun une liaison, L¹ est l'oxygène, Q² et Z² sont tous deux hydrogène, k = j = 1, X¹ et X² sont l'iodine, et au moins l'un de y1 et y2 est non nul.

14. Composition polymère comprenant un polymère biocompatible selon l'une quelconque des revendications 1 à 11.

15. Dispositif médical, de préférence un stent, comprenant le polymère biocompatible selon l'une quelconque des revendications 1 à 11.
